# EUROPÄISCHE PATENTSCHRIFT

(11) **EP 3 372 306 B1**
(45) Veröffentlichungstag und Bekanntmachung des Hinweises auf die Patenterteilung: **01.04.2020**
(21) Anmeldenummer: 18158012.7
(22) Anmeldetag: 22.02.2018
(51) Int. Cl.: B01F 13/00, B01F 15/02, A61B 17/88

(54) **ZWEITEILIGE LAGER- UND MISCHVORRICHTUNG ZUR HERSTELLUNG EINES KNOCHENZEMENTS UND VERFAHREN HIERZU**
TWO-PART STORAGE AND MIXING DEVICE FOR THE PRODUCTION OF A BONE CEMENT, AND PERTINENT METHOD
DISPOSITIF DE STOCKAGE ET DE MÉLANGE EN DEUX PARTIES DESTINÉ À LA PRODUCTION D'UN CIMENT OSSEUX ET PROCÉDÉ CORRESPONDANT

(30) Priorität: 08.03.2017 DE 102017104854
(43) Veröffentlichungstag der Anmeldung: 12.09.2018
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- EP-A1- 0 692 229
- US-A- 4 463 875

## Beschreibung

Die Erfindung betrifft eine Lager- und Mischvorrichtung zur Herstellung eines Knochenzementteigs aus einer Monomerflüssigkeit und einem Zementpulver als Ausgangskomponenten des Knochenzements.

Die Erfindung betrifft auch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Lager- und Mischvorrichtung hergestellt wird.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Der mit der Vorrichtung hergestellte Knochenzementteig ist insbesondere für die Augmentation von frakturierten Wirbelkörpern bestimmt, also für die Vertebroplastie. Es handelt sich bei der erfindungsgemäßen Lager- und Mischvorrichtung um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Für die Behandlung von Impressionsfrakturen von Wirbelkörpern wurde eine Reihe von speziellen Polymethylmethacrylat-Knochenzementen entwickelt. Diese zeichnen sich dadurch aus, dass sie einen relativ hohen Anteil an Röntgenopakern, zum Beispiel Zirkoniumdioxid oder Bariumsulfat, enthalten. Dadurch soll eine fortlaufende Kontrolle der Ausbreitung des Knochenzementteigs im frakturierten Wirbel durch Fluoroskopie erleichtert werden. Die gegenwärtig hauptsächlich angewandten Verfahren zur Augmentation von frakturierten Wirbelkörpern sind die Vertebroplastie und die Kyphoplastie. Bisher ist dabei die manuelle Vermischung der Zementkomponenten in Mischbechern oder in einfachen Mischvorrichtungen üblich.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Aus der EP 2 730 296 A2 ist ein thixotroper Knochenzement für die Vertebroplastie bekannt, bei dem mit mehreren Additiven die thixotropen Eigenschaften erzeugt werden.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird bei dem geschlossenen Zementiersystem PALACOS® PRO verwirklicht, das von der Firma Heraeus Medical GmbH hergestellt und vertrieben wird.

Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischvorrichtungen befindet sich der Zementteig im Fall von Pulver-Flüssigkeits-Zementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Auspressvorrichtung beim Auspressvorgang angreift, eine Fläche von 7,0 cm² bis 12,5 cm². Die Bewegung des Austragskolbens wird durch manuell bedienbare, mechanische Auspressvorrichtungen bewirkt, die auch als Applikatoren bezeichnet werden. Diese manuellen Auspressvorrichtungen erreichen normalerweise eine Auspresskraft im Bereich von ungefähr 1,5 kN bis 3,5 N.

Bei der Applikation konventioneller PMMA-Knochenzemente, die aus einer flüssigen Monomerkomponente und einer separat dazu gelagerten Zementpulverkomponente als Ausgangskomponenten bestehen, wird nach der Vermischung der beiden Ausgangskomponenten in Zementiersystemen der gebildete Zementteig mit Hilfe von manuell bedienbaren Auspressvorrichtungen ausgepresst. Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Mischvorrichtungen muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

Die US 4 463 875 offenbart ein System zum Mischen eines Knochenzements, bei dem eine Mischung eines Zementpulvers mit einer Monomerflüssigkeit durch abwechselndes Bedienen zweiteiliger Stößel von beiden Richtung in einen Folienbehälter für das Zementpulver und einen Folienbehälter für die Monomerflüssigkeit hergestellt wird.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei hochviskosen pastenförmigen Knochenzementen unter Verwendung von Kartuschen, bei denen der Austragskolben an der äußeren Kolbenseite, an der ein Stößel der Auspressvorrichtungen angreift, eine gesamte Fläche im Bereich von 7,0 cm² bis 12,5 cm² hat, sind solche Vorrichtungen nicht oder nur mit einem sehr großen Kraftaufwand manuell zu bedienen. Dies gilt insbesondere dann, wenn der Fließwiderstand des auszupressenden Knochenzementteigs durch eine verlängerte Austragsöffnung und/oder durch einen statischen Mischer erhöht wird, wie dies bei Anwendungen im Bereich der Wirbelsäule, wo der Knochenzementteig über einen Schlauch und einen Trokar ausgetragen wird, üblich ist. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der Vertebroplastie wird die Applikation von Knochenzement mit einem Röntgenverfahren in situ verfolgt. Bei Applikationsvorrichtungen für die Vertebroplastie wird üblicherweise ein Schlauch eingesetzt, über dessen Spitze der Knochenzement appliziert werden kann, damit der Anwender außerhalb des Bereichs der Röntgenstrahlung arbeiten kann. Hierzu können am Schlauch des Weiteren ein Trokar oder eine Kanüle angeordnet sein. Derartige Systeme sind beispielsweise aus US 7 112 205 B2, US 8 038 682 B2, US 8 308 731 B2, DE 10 2005 045 227 A1, EP 1 074 231 B1, EP 1 596 736 B1, US 9 005 209 B2 und WO 2008/097855 A2 bekannt.

Alternativ können auch andere Aufbauten verwendet werden, um den Anwender von der Röntgenstrahlung fernzuhalten, wie beispielsweise in den Dokumenten US 6 676 663 B2, US 7 008 433 B2, US 8 348 494 B2, EP1 464 292 B1, EP 1 614 403 B1, US 2008/319445 A9 und WO 2008/038322 A2 beschrieben.

Ein Knochenzement-Applikator für die Vertebroplastie zum Applizieren von Knochenzement mit einem Schlauch, einem Trokar und einem Mischer ist aus der US 2008/0086143 A1 bekannt. Der Knochenzement-Applikator umfasst zwei nebeneinander angeordnete Kartuschen, in denen die Ausgangskomponenten auch gelagert werden. Der Knochenzement-Applikator wird unmittelbar vor der Anwendung zusammengesetzt. Bei derartigen Knochenzement-Applikatoren für die Vertebroplastie wird auf die Ausgangskomponenten des Knochenzements mit Hilfe einer Auspressvorrichtung, die Austragskolben in den Kartuschen vortreiben, ein Druck ausgeübt und mit Hilfe des Drucks die Ausgangskomponenten aus den Kartuschen und durch den Schlauch ausgetrieben. Dabei werden die Ausgangskomponenten meist zuvor in einem vorgeschalteten statischen Mischer gemischt. Dies führt dazu, dass sich die den Knochenzementfluss begrenzenden Teile des Knochenzement-Applikators (die Kartuschen, das Gehäuse des Mischers und der Schlauch) elastisch verformen. Wenn der Vortrieb der Austragskolben gestoppt wird, kommt es durch die elastische Kraft dieser Teile zu einer Volumenkontraktion dieser Teile, so dass auch dann noch Knochenzement durch die Applikationsöffnung des Schlauchs beziehungsweise Trokars austritt. Hierdurch kann es zu einer Kontamination des OP-Saals oder des Anwenders mit dem Knochenzement kommen oder es wird ungewollt eine zu große Menge des Knochenzements appliziert. Zudem muss, wenn der Volumenstrom des Knochenzementteigs wieder gestartet werden soll, zunächst wieder der Druck im Knochenzement aufgebaut werden, damit dieser durch die Applikationsöffnung austritt. Dadurch verzögert sich wiederum der Zeitpunkt nach dem Beginn des Vortreibens der Austragskolben, ab dem tatsächlich der Knochenzementteig appliziert werden kann, was ebenfalls unerwünscht ist. Aufgrund der großen Zähigkeit des Knochenzementteigs und der Ausgangskomponenten, insbesondere, wenn pastöse Ausgangskomponenten verwendet werden, sind alle diese Effekte relativ stark ausgeprägt. Dem kann durch die Verwendung massiver und teurer metallisches Gehäuseteile entgegengewirkt werden. Diese Teile müssen nach der Anwendung gereinigt und für eine weitere Anwendung sterilisiert werden oder sie müssen aufwendig recycelt werden. Zudem können beim Lösen der Kartuschen Reste der Ausgangskomponenten freigesetzt werden und dadurch den OP-Saal kontaminieren.

Die US 8,544,683 B2 offenbart ein Kartuschensystem, das zur Beimischen einer geringen Menge zu einer Hauptausgangskomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Ausgangskomponenten von PMMA-Knochenzement jedoch nicht geeignet.

Die Aufgabe der Erfindung besteht also darin, die Nachteile des Stands der Technik zu überwinden. Insbesondere besteht die Aufgabe der Erfindung in der Entwicklung einer Lager- und Mischvorrichtung und eines Verfahrens, die beide zum Vermischen und anschließend zum Austragen und vorzugsweise auch zum Lagern von einem Zementpulver und einer Monomerflüssigkeit als Ausgangskomponenten eines Polymethylmethacrylat-Knochenzements geeignet sind. Zudem soll ein Verfahren zur Herstellung eines Prepacked-PMMA-Zementkartuschensystems, das zum Mischen und Applizieren eines PMMA-Knochenzementteigs geeignet ist, bereitgestellt werden, mit dem die Probleme überwunden werden. Die Lager- und Mischvorrichtung soll durch eine herkömmliche Auspresseinrichtung anzutreiben sein und dabei möglichst einfach in der Bedienung sein. Der Aufbau soll kostengünstig sein, damit die Lager- und Mischvorrichtung aus hygienischen Gründen nur einmalig verwendet werden kann. Es sollen möglichst viele oder alle in der Lager- und Mischvorrichtung ablaufenden Prozesse, wie die Vermischung der Ausgangskomponenten, das Austragen des Knochenzementteigs und das Öffnen von Behältern und gegebenenfalls der Kartusche, mit möglichst wenigen Arbeitsschritten und so weit als möglich automatisiert ablaufen und vorzugsweise mit einem einzigen Antrieb anzutreiben sein.

Bevorzugt soll auch ein einfacher und kostengünstig zu fertigender Knochenzement-Applikator für die Vertebroplastie für pastenförmige Mehrkomponenten-Polymethylmethacrylat-Knochenzemente und ein Verfahren zum Applizieren eines Zementteigs mit einer Lager- und Mischvorrichtung bereitgestellt werden, der einfach aufgebaut ist und kostengünstig zu fertigen ist, wobei es beim Stoppen des Zementflusses nicht zu einem Nachlaufen des Knochenzementteigs kommen soll. Ferner soll die Lager- und Mischvorrichtung nach Unterbrechung des Flusses des Knochenzementteigs möglichst schnell wieder einsetzbar sein. Es soll eine Kontamination der Umgebung und des Anwenders mit Knochenzementteig oder den Ausgangskomponenten, insbesondere der Monomerflüssigkeit möglichst ausgeschlossen werden.

Die Aufgabe der Erfindung besteht also insbesondere in der Entwicklung einer Lager- und Mischvorrichtung zum Lagern und Vermischen von Zementpulver und Monomerflüssigkeit, wobei der durch Vermischung der Ausgangskomponenten gebildete Polymethylmethacrylat-Knochenzementteig bevorzugt zur Augmentation von frakturierten Wirbelkörpern bestimmt ist. Die Handhabung der Lager- und Mischvorrichtung soll maximal vereinfacht sein, um Anwendungsfehler infolge von fehlerhaft durchgeführten Montageschritten grundsätzlich zu vermeiden. Der medizinische Anwender soll die Lager- und Mischvorrichtung nach der Entnahme aus einer Verpackung mit einer Auspressvorrichtung verbinden und diese anschließend manuell betätigen können. Die Lager- und Mischvorrichtung soll eine sichere Lagerung von Zementpulver und Monomerflüssigkeit in voneinander getrennten Kompartimenten ermöglichen, so dass während der Lagerung der Lager- und Mischvorrichtung eine unbeabsichtigte Vermischung der Zementkomponenten ausgeschlossen ist. Die Lager- und Mischvorrichtung soll eine Sterilisation mit dem Gas Ethylenoxid ermöglichen. Das in der Lager- und Mischvorrichtung gelagerte Zementpulver muss für Ethylenoxid zugänglich sein. Die Lager- und Mischvorrichtung soll mit Hilfe einer im OP bisher üblichen manuell angetriebenen Auspressvorrichtung aktivierbar sein, so dass nach der form- oder kraftschlüssigen Verbindung der Lager- und Mischvorrichtung oder Teilen davon mit der Auspressvorrichtung durch Betätigung der Auspressvorrichtung der Stößel der Auspressvorrichtung auf die Lager- und Mischvorrichtung einwirkt und den Monomerflüssigkeitsbehälter öffnet und anschließend bei weiterer Bewegung des Stempels die Monomerflüssigkeit in das Zementpulver transferiert. Die Vermischung der Monomerflüssigkeit mit dem Zementpulver soll ohne einen von außen manuell zu bewegenden Mischer erfolgen. Es soll nur mit der Vorwärtsbewegung des Stößels der Auspressvorrichtung das Öffnen des Monomerflüssigkeitsbehälters, der nachfolgende Monomertransfer in das Zementpulver und die Vermischung der Zementkomponenten unter Bildung des Zementteigs vorgenommen werden. Es ist wichtig, dass der Austragskolben zum Austrag des gebildeten Zementteigs so gestaltet ist, dass bei der Verwendung einer üblichen, manuellen Auspressvorrichtung der Druck auf den Austragskolben so hoch ist, dass der Zementteig durch einen Kunststoffschlauch mit einem Innendurchmesser von 3 mm mindestens über eine Strecke von 20 cm ausgepresst werden kann.

Die Lager- und Mischvorrichtung soll möglichst einfach aus Kunststoff gefertigt werden können und sich dadurch als Produkt zur einmaligen Anwendung eignen. Das Auspressen des gemischten Zementteigs soll mit einer konventionellen, manuell angetriebenen Auspressvorrichtung, wie sie bisher bei PMMA-Knochenzementen zur Zementierung von Knie- und Hüft-TEP (totale Endoprothese des Hüftgelenks) üblich ist, möglich sein. Der Knochenzement-Applikator soll so beschaffen sein, dass ein sofortiger Notstop des fließenden Knochenzementteigs möglich ist, ohne dass eine Kontamination des OPs (Operationssaals) mit dem Knochenzementteig beziehungsweise ein Weiterfließen des Knochenzementteigs erfolgt.

Bevorzugt soll der zu entwickelnde Knochenzement-Applikator keine zwei miteinander verbundenen und synchron vorzutreibenden Stößel erfordern, damit die gesamte Vorrichtung nicht wesentlich aufwendiger, länger und größer wird als die bisher für die konventionellen Pulver-Flüssigkeits-Polymethylmethacrylat-Knochenzemente üblichen Mischvorrichtungen. Es soll eine einfache Lösung gefunden werden, die es erlaubt, möglichst mit nur einem Stößel und gegebenenfalls einem daran befestigten Teller den Knochenzementteig auszutreiben.

Die Aufgaben der Erfindung werden gelöst durch eine Lager- und Mischvorrichtung zur Herstellung eines Knochenzementteigs aus einer Monomerflüssigkeit und einem Zementpulver als Ausgangskomponenten des Knochenzements, die Lager- und Mischvorrichtung aufweisend
A) eine Aufnahme, in der ein Monomerflüssigkeitsbehälter angeordnet ist, wobei in dem Monomerflüssigkeitsbehälter die Monomerflüssigkeit enthalten ist, und
B) eine Kartusche, in der das Zementpulver enthalten ist, wobei
C) in der Aufnahme ein in Längsrichtung der Aufnahme beweglicher Förderkolben angeordnet ist, der von einer Rückseite der Aufnahme aus zugänglich ist, wobei
D) in der Kartusche ein in Längsrichtung im Inneren der Kartusche verschiebbarer Austragskolben angeordnet ist, wobei
E) die Vorderseite der Aufnahme mit der Rückseite der Kartusche derart verbunden oder verbindbar ist, dass ein Innenraum der Aufnahme mit einem Innenraum der Kartusche für die Monomerflüssigkeit durchlässig verbunden ist, wobei die Verbindung lösbar ist, und wobei
F) an der Rückseite der Aufnahme ein erstes Befestigungsmittel vorgesehen ist und an der Rückseite der Kartusche ein zweites Befestigungsmittel vorgesehen ist, wobei das erste Befestigungsmittel und das zweite Befestigungsmittel gleich oder gleichartig sind, wobei an der Rückseite der Kartusche, insbesondere im Austragskolben der Kartusche, eine verschließbare Durchführung in den Innenraum der Kartusche vorgesehen ist.

Vorzugsweise sind das erste Befestigungsmittel und das zweite Befestigungsmittel gleich, also identisch. Dadurch können sowohl die Aufnahme als auch die Kartusche in gleicher Weise an der gleichen Halterung einer Auspressvorrichtung, wie einer Kartuschenpistole, befestigt werden und damit die Aufnahme und die Kartusche mit der gleichen Auspressvorrichtung ausgepresst werden. Unter dem Begriff Kartuschenpistole werden auch Kartuschenpressen verstanden. Vorzugsweise wird eine manuell angetriebene Auspressvorrichtung oder Kartuschenpistole verwendet.

Die Lager- und Mischvorrichtung ist erfindungsgemäß bevorzugt zweiteilig.

Es kann also bevorzugt vorgesehen sein, dass das erste Befestigungsmittel und das zweite Befestigungsmittel zur Befestigung an einer Halterung einer Auspressvorrichtung geeignet und vorgesehen sind.

Als Knochenzement wird vorzugsweise ein Polymethylmethacrylat-Knochenzement (PMMA-Knochenzement) verwendet. Das Zementpulver ist dann ein PMMA-Zementpulver.

Bevorzugt kann vorgesehen sein, dass der Austragskolben von einer Rückseite der Kartusche zugänglich ist, wenn die Aufnahme und die Kartusche nicht miteinander verbunden sind.

Vorzugsweise ist der Monomerflüssigkeitsbehälter ein zur Aufnahme separates Behältnis.

Es kann auch vorgesehen sein, dass an der Vorderseite der Kartusche eine Austragsöffnung, insbesondere als Teil eines Austragsrohrs, zum Austragen des Knochenzementteigs vorgesehen ist. Dabei kann bevorzugt vorgesehen sein, dass ein abnehmbarer oder zu öffnender, gasdurchlässiger Verschluss die Austragsöffnung für Zementpulverpartikel verschließt. Dabei kann wiederum bevorzugt vorgesehen sein, dass der Verschluss durch axialen Druck oder durch manuelle Krafteinwirkung zu öffnen ist.

Bei Lager- und Mischvorrichtung kann erfindungsgemäß vorgesehen sein, dass die verschließbare Durchführung beim Lösen der flüssigkeitsdurchlässigen Verbindung zwischen der Aufnahme und der Kartusche flüssigkeitsdicht verschließbar ist, wobei besonders bevorzugt die verschließbare Durchführung ein Ventil, zumindest zwei Dichtlippen oder eine selbstschließende durchstechbare Membran aufweist.

Durch die Verschließbarkeit der Durchführung kann erreicht werden, dass nachdem die Monomerflüssigkeit in die Kartusche eingetragen wurde, durch diese keine Monomerflüssigkeit nach dem Lösen der Aufnahme von der Kartusche an der Rückseite der Kartusche austreten kann. Hierdurch wird eine Kontamination beziehungsweise Verschmutzung des OP-Bereichs mit der Monomerflüssigkeit verhindert.

Bevorzugt kann dabei vorgesehen sein, dass in der verschließbaren Durchführung ein für das Zementpulver undurchlässiger und für die Monomerflüssigkeit durchlässiger Filter, insbesondere ein Porenfilter, angeordnet ist. Hierdurch kann verhindert werden, dass das Zementpulver in die verschließbare Durchführung eindringt, dort mit der Monomerflüssigkeit reagiert, dort quillt und dadurch die Durchführung ungewollt verschließt.

Ferner kann vorgesehen sein, dass an der Vorderseite der Aufnahme eine Hohlnadel oder ein Röhrchen vorgesehen ist, mit dem das Innere der Aufnahme flüssigkeitsleitend mit dem Innenraum der Kartusche verbindbar ist, wobei die Hohlnadel oder das Röhrchen mit der verschließbaren Durchführung die für die Monomerflüssigkeit durchlässige Verbindung in den Innenraum der Kartusche bildet, wenn die Aufnahme mit der Kartusche verbunden ist.

Hiermit wird erreicht, dass über die Hohlnadel oder das Röhrchen der Verschluss der verschließbaren Durchführung auf einfache und sichere Weise geöffnet werden kann und dieser sich wieder selbsttätig schließt, wenn die Hohlnadel oder das Röhrchen aus der verschließbaren Durchführung herausgezogen werden. Selbstverständlich können auch mehrere Hohlnadeln oder mehrere Röhrchen hierzu verwendet werden.

Es wird mit der vorliegenden Erfindung auch vorgeschlagen, dass an der Vorderseite der Aufnahme ein Verbindungskolben angeordnet ist, der in Längsrichtung beweglich im Inneren der Aufnahme angeordnet ist, wobei der Monomerflüssigkeitsbehälter zwischen dem Verbindungskolben und dem Förderkolben angeordnet ist, wobei in dem Verbindungskolben ein Durchgang vorgesehen ist, der für die Monomerflüssigkeit und für Gase durchlässig ist und mit dem eine Flüssigkeitsverbindung zu dem Innenraum der Kartusche herstellbar ist, wobei vorzugsweise durch eine Bewegung des Verbindungskolbens in Richtung der Kartusche eine mit der Vorderseite der Aufnahme verbundenen Kartusche, die verschließbare Durchführung an der Rückseite der Kartusche oder ein Verschlussmittel, das die flüssigkeitsdurchlässige Verbindung zwischen dem Innenraum der Kartusche und dem Innenraum der Aufnahme verschließt, zu öffnen ist.

Hiermit wird erreicht, dass die Flüssigkeitsverbindung zwischen dem Innenraum der Kartusche und dem Innenraum der Aufnahme erst durch eine Bewegung des Verbindungskolbens geöffnet wird oder die Lager- und Mischvorrichtung leichter und einfacher aufzubauen ist. Durch den beweglichen Verbindungskolben kann eine ungewollte Öffnung des Innenraums der Kartusche vermieden werden. Des Weiteren kann durch die Bewegung des Verbindungskolbens die verschließbare Durchführung geöffnet werden, so dass die Öffnung des Verschlusses der verschließbaren Durchführung durch den gleichen linearen Antrieb erfolgen kann, der auch zum Antreiben des Förderkolbens und des Austragskolbens verwendet wird.

Vorzugsweise ist in dem Verbindungskolben die Hohlnadel oder das Röhrchen angeordnet. Dabei kann bevorzugt vorgesehen sein, dass im Lagerzustand der Lager- und Mischvorrichtung der Verbindungskolben in einem solchen Abstand vom Austragskolben oder der Kartusche in der Aufnahme angeordnet ist, dass die Hohlnadel oder das Röhrchen den Verschluss des verschließbaren Durchgangs an der Rückseite der Kartusche nicht berührt. Wobei vorzugsweise die Hohlnadel eine durchstechbare Membran des verschließbaren Durchgangs nicht berührt oder das Röhrchen die Dichtlippen oder das Ventil des verschließbaren Durchgangs nicht berührt. Bei einem Aufbau mit Dichtlippen ist das Röhrchen allerdings bevorzugt bereits durch die Dichtlippen geführt.

Des Weiteren kann vorgesehen sein, dass an der Vorderseite in der Aufnahme ein Anschlag vorgesehen ist, der die Bewegung des Verbindungskolbens in Richtung der Kartusche oder die Bewegung des Verbindungskolbens in Richtung der Vorderseite der Aufnahme begrenzt.

Gemäß einer bevorzugten Weiterbildung kann vorgesehen sein, dass an der Vorderseite der Aufnahme in der flüssigkeitsdurchlässigen Verbindung zur Kartusche ein Sieb oder eine für Gase und Flüssigkeiten durchlässige poröse Scheibe vorgesehen ist.

Hierdurch kann verhindert werden, dass Bruchstücke oder abgetrennte Stücke des Monomerflüssigkeitsbehälters in die Kartusche und damit in den Knochenzement gelangen. Zudem kann so auch verhindert werden, dass solche Bruchstücke oder Stücke die flüssigkeitsdurchlässige Verbindung zwischen der Aufnahme und der Kartusche beeinträchtigen oder blockieren.

Es kann auch vorgesehen sein, dass die Lager- und Mischvorrichtung ein Knochenzement-Applikator zum Lagern und Mischen der Ausgangskomponenten und zur Applikation des Knochenzementteigs im Bereich der Wirbelsäule ist, wobei bevorzugt der Knochenzement-Applikator eine verlängerte Austragsöffnung aufweist, die an der Vorderseite der Kartusche angeordnet ist, wobei besonders bevorzugt die verlängerte Austragsöffnung durch ein Austragsrohr, einen Schlauch und/oder einen Trokar realisiert ist.

Die Lager- und Mischvorrichtung lässt sich besonders gut als Knochenzement-Applikator für die Vertebroplastie einsetzen. Durch den linearen zweiteiligen Aufbau und durch die Möglichkeit, die Kartusche sehr schmal zu gestalten, kann trotz des sehr zähen Knochenzementteigs und einer langen Austragsöffnung, gegebenenfalls mit Trokar und Schlauch, durch die der Fließwiderstand zusätzlich erhöht wird, eine manuell angetriebene Auspressvorrichtung verwendet werden. Die Hubstrecke, mit dem die Lager- und Mischvorrichtung und damit der Knochenzement-Applikator angetrieben wird, ist nämlich sehr lang und durch den gestreckten Aufbau und den damit einhergehenden relativ geringen Innendurchmesser des Innenraums der Kartusche wird durch einen langen Hub nur eine geringe Menge des Knochenzementteigs durch die verlängerte Austragsöffnung gepresst.

Vorzugsweise ist die verlängerte Austragsöffnung durch ein Austragsrohr realisiert, an dem ein Adapter, insbesondere ein Luer-System-Adapter, zur Befestigung eines Schlauchs oder eines Trokars angeordnet ist. Bevorzugt hat der Schlauch einen Innendurchmesser kleiner als 4 mm. Ebenfalls bevorzugt hat der Schlauch eine Mindestdruckfestigkeit von 10 bar.

Bei solchen als Knochenzement-Applikatoren für die Wirbelversteifung vorgesehenen Lager- und Mischvorrichtungen kann vorgesehen sein, dass ein von außen bedienbares Dreiwege-Ventil in der verlängerten Austragsöffnung oder zwischen der verlängerten Austragsöffnung und der Kartusche angeordnet ist, ein Auffangbehälter zum Aufnehmen von Knochenzementteig an dem Dreiwege-Ventil angeordnet ist, wobei die verlängerte Austragsöffnung in eine Applikationsöffnung mündet, die an dem von der Kartusche abgewandten Ende der verlängerten Austragsöffnung angeordnet ist, wobei das Dreiwege-Ventil derart aufgebaut ist, dass es in einer ersten Stellung eine Fluidverbindung zwischen der Applikationsöffnung und der Kartusche bereitstellt und einen Ablasskanal zum Auffangbehälter verschließt und in einer zweiten Stellung eine Fluidverbindung zwischen der Applikationsöffnung und dem Auffangbehälter bereitstellt und einen Durchgang zur Kartusche verschließt.

Dadurch kann erreicht werden, dass bei einer Unterbrechung des Auspressvorgangs und durch Einstellen eines Dreiwege-Ventils, das mit dem Auffangbehälter, dem Schlauch und der Kartusche verbunden ist, der Druck von dem Knochenzement in einer verlängerten Austragsöffnung, wie einem Schlauch, genommen werden kann, ohne dass eine größere Menge des Knochenzementteigs nachfließt. Gleichzeitig kann auf diese Weise der Druck des Knochenzementteigs und der Ausgangskomponenten in der Kartusche bis zum Dreiwege-Ventil und insbesondere auch im Mischer, sofern vorhanden, aufrechterhalten werden. Dadurch ist die Zeit, die nach dem Öffnen des Dreiwege-Ventils bis zum erneuten Austritt des Knochenzementteigs vergeht, sehr kurz. Die Spannung der Kartusche wird zwischen dem Dreiwege-Ventil und dem Austragskolben also gehalten, wenn das Dreiwege-Ventil geschlossen ist, während eine schnelle Entspannung der verlängerten Austragsöffnung zwischen dem Dreiwege-Ventil und der Applikationsöffnung durch Abfließen des Knochenzementteigs durch das Dreiwege-Ventil in der geschlossenen Stellung erreicht wird. Damit der Knochenzementteig nicht die Umgebung oder den Anwender kontaminiert, ist der Auffangbehälter vorgesehen, der ein Abtropfen des durch das Dreiwege-Ventil austretenden Knochenzementteigs verhindert. Theoretisch kann es ausreichen, wenn der Knochenzementteig zurückgehalten wird. Der Auffangbehälter kann auch flexibel und/oder elastisch sein und sich bei der Aufnahme des aus dem Dreiwege-Ventil austretenden Knochenzementteigs ausdehnen.

Die Lager- und Mischvorrichtung enthält so ein Notablassventil in Form des Dreiwege-Ventils, mit dem der Auspressvorgang sofort gestoppt werden kann, wenn der Knochenzementteig in unerwünschte Bereiche des Wirbelkörpers zu fließen beginnt. Dieses Notablassventil druckentlastet die verlängerte Austragsöffnung der Lager- und Mischvorrichtung, in dem der Nachdruck des Knochenzementteigs aus den Bereichen davor blockiert wird und gleichzeitig der vor dem Notablassventil befindliche Knochenzementteig druckentlastet wird, in dem ein Kanal in einen Auffangbehälter geöffnet wird, in den der Knochenzementteig austreten kann bis der Druck im Schlauch beziehungsweise im Trokar abgebaut ist.

Bei derartigen Lager- und Mischvorrichtungen kann bevorzugt vorgesehen sein, dass der Auffangbehälter nach außen für den Knochenzementteig undurchlässig ist, vorzugsweise der Auffangbehälter flüssigkeitsdicht oder flüssigkeitsdicht und gasdicht ist. Es kann auch vorgesehen sein, dass der Auffangbehälter ein Volumen aufweist, das mindestens so groß ist wie die Hälfte des Volumens der verlängerten Austragsöffnung, insbesondere des Schlauchs und gegebenenfalls des Trokars, bevorzugt mindestens so groß ist wie das Volumen der verlängerten Austragsöffnung, insbesondere des Schlauchs und gegebenenfalls des Trokars.

Es kann erfindungsgemäß vorgesehen sein, dass zwischen der Kartusche und der verlängerten Austragsöffnung oder in der verlängerten Austragsöffnung oder zwischen der Kartusche und dem Dreiwege-Ventil ein Mischer zum Mischen des Knochenzements, insbesondere ein statischer Mischer, angeordnet ist, wobei vorzugsweise das Dreiwege-Ventil zwischen dem Mischer und der verlängerten Austragsöffnung angeordnet ist. In letzterem Fall kann vorgesehen sein, dass das Dreiwege-Ventil in der ersten Stellung eine Fluidverbindung zwischen der Applikationsöffnung und dem Mischer bereitstellt und in der zweiten Stellung den Durchgang zum Mischer verschließt.

Es kann bei erfindungsgemäßen Lager- und Mischvorrichtungen mit Schlauch auch vorgesehen sein, dass der Schlauch zumindest bereichsweise flexibel ist. Es kann auch vorgesehen sein, dass die verlängerte Austragsöffnung in einem Anschluss mit einem Innengewinde, insbesondere in einem Luer-System-Adapter, oder in einem Trokar endet.

Bevorzugte Lager- und Mischvorrichtungen können sich auch dadurch auszeichnen, dass die Aufnahme ein Verbindungsmittel aufweist, insbesondere zumindest eine Hohlnadel oder zumindest ein Röhrchen als Verbindungsmittel aufweist, wobei das Verbindungsmittel an der Vorderseite der Aufnahme angeordnet ist und die Aufnahme über das Verbindungsmittel flüssigkeitsleitend mit dem Innenraum der Kartusche verbunden oder verbindbar ist und wobei die Aufnahme bis auf das Verbindungsmittel flüssigkeitsdicht geschlossen ist.

Hierdurch kann gezielt eine Verbindung zwischen der Aufnahme und der Kartusche durch die Aufnahme erreicht werden. Zudem wird das Verbindungsmittel mit dem Entfernen der Aufnahme von der Kartusche entfernt und kann somit nachfolgend nicht mehr beim Austreiben des Knochenzements aus der Kartusche stören.

Es kann des Weiteren vorgesehen sein, dass der Monomerflüssigkeitsbehälter im Inneren der Aufnahme durch eine Bewegung des Förderkolbens in Richtung der Vorderseite der Aufnahme zu öffnen ist, bevorzugt aufzubrechen oder aufzureißen ist.

Hierdurch wird erreicht, dass durch die axiale lineare Bewegung des Förderkolbens der Monomerflüssigkeitsbehälter geöffnet werden kann. Es kann dadurch eine Auspressvorrichtung mit nur einem axialen linearen Antrieb verwendet werden, um sowohl den Monomerflüssigkeitsbehälter zu öffnen als auch die Monomerflüssigkeit in die Kartusche zu pressen und auch den Knochenzementteig aus der Kartusche zu pressen.

Gemäß einer Weiterbildung der erfindungsgemäßen Lager- und Mischvorrichtung kann auch vorgesehen sein, dass der Monomerflüssigkeitsbehälter eine Glasampulle, eine Kunststoffampulle ein Kunststoff-Folienbeutel oder ein Aluminium-Kunststoff-Verbund-Beutel ist, die oder der im Inneren der Aufnahme zu öffnen ist.

Derartige Monomerflüssigkeitsbehälter sind zur langfristigen Lagerung von Monomerflüssigkeit gut geeignet und lassen sich auch gut in der Aufnahme aufbrechen oder aufreißen.

Bevorzugt kann vorgesehen sein, dass das erste Befestigungsmittel und das zweite Befestigungsmittel an einer einzigen Halterung einer Auspressvorrichtung, insbesondere einer Kartuschenpistole, zu befestigen sind.

Hierdurch kann eine einzige Auspressvorrichtung dazu verwendet werden, um sowohl den Förderkolben als auch den Austragskolben anzutreiben. Die Bedienung der Lager- und Mischvorrichtung wird dadurch vereinfacht.

Ebenso kann bevorzugt vorgesehen sein, dass der Innenraum der Kartusche zylindrisch ist und dass der Austragskolben einen zu dem Innenraum der Kartusche passenden äußeren Umfang aufweist, so dass der Austragskolben den Innenraum der Kartusche an dessen Rückseite nach außen abdichtet.

Hierdurch wird der Aufbau der Lager- und Mischvorrichtung vereinfacht und kostengünstig gestaltet. Zudem kann der Austragskolben bei dieser Geometrie nicht so leicht im Innenraum der Kartusche verkanten, so dass die Lager- und Mischvorrichtung hierdurch für Fehler unanfälliger wird.

Die Kartusche ist vorzugsweise röhrenförmig.

Des Weiteren kann vorgesehen sein, dass der Innenraum der Aufnahme zylindrisch ist und dass der Förderkolben einen zu dem Innenraum der Aufnahme passenden äußeren Umfang aufweist, so dass der Förderkolben den Innenraum der Aufnahme an deren Rückseite nach außen abdichtet.

Hierdurch wird der Aufbau der Lager- und Mischvorrichtung vereinfacht und kostengünstig gestaltet. Zudem kann der Förderkolben bei dieser Geometrie nicht so leicht im Innenraum der Aufnahme verkanten, so dass die Lager- und Mischvorrichtung hierdurch für Fehler unanfälliger wird.

Die Aufnahme ist vorzugsweise röhrenförmig.

Es kann auch vorgesehen sein, dass an der Vorderseite der Kartusche eine Austragsöffnung vorgesehen ist, durch die der Inhalt der Kartusche mit dem Austragskolben austreibbar ist.

Hiermit wird eine Austragsöffnung an der dem Austragskolben gegenüberliegenden Seite bereitgestellt. Hierdurch wird der Strömungswiderstand für den zähflüssigen Knochenzementteig gering gehalten.

Um die Lager- und Mischvorrichtung unabhängig von externer Versorgung antreiben zu können, kann vorgesehen sein, dass die Lager- und Mischvorrichtung mit Hilfe einer manuell bedienbaren Auspressvorrichtung bedienbar ist und der Austragskolben mit manueller Kraft in der Kartusche bewegbar ist, so dass der Knochenzementteig auszutreiben ist, und der Förderkolben mit manueller Kraft in der Aufnahme bewegbar ist, so dass der Monomerflüssigkeitsbehälter zu öffnen und die Monomerflüssigkeit in den Innenraum der Kartusche zu pressen ist.

Hierdurch ist die Lager- und Mischvorrichtung auch ohne Motoren und externe Energiequellen zuverlässig einsetzbar.

Ferner kann vorgesehen sein, dass der Querschnitt des Innenraums der Kartusche maximal 4 cm² beträgt, bevorzugt maximal 2,5 cm² beträgt, besonders bevorzugt maximal 1,2 cm² beträgt.

Hiermit kann sichergestellt werden, dass die im Inneren der Kartusche auftretenden Kräfte bei Austreiben des zähen Knochenzementteigs nicht zu groß werden. Hierdurch kann einerseits der Aufbau vereinfacht werden und andererseits kann eine manuell angetriebene Auspressvorrichtung verwendet werden.

Mit einer bevorzugten Weiterbildung kann vorgesehen sein, dass die Kartusche an der Vorderseite mit einem Kartuschenkopf abgeschlossen ist, wobei sich im Kartuschenkopf eine Auslassöffnung befindet und die Auslassöffnung mit einem für das Zementpulver in der Kartusche undurchlässigen und für Gas durchlässigen Verschluss, insbesondere einem Porenfilter, verschlossen ist, wobei vorzugsweise der Verschluss durch axiale Druckbeanspruchung oder durch manuelle Krafteinwirkung zu öffnen ist.

Hierdurch können das Innere der Kartusche und das Zementpulver mit Hilfe eines sterilisierenden Gases wie Ethylenoxid sterilisiert werden. Gleichzeitig kann das Zementpulver nicht nach außen dringen und verloren gehen, wodurch ansonsten das Mischungsverhältnis Zementpulver zu Monomerflüssigkeit und damit die Zusammensetzung des Knochenzementteigs verfälscht würde.

Zur langfristigen Lagerung des Zementpulvers kann vorgesehen sein, dass der Austragskolben für die Partikel des Zementpulvers undurchlässig ist und einen für die Monomerflüssigkeit durchlässige Durchführung aufweist, wobei vorzugsweise die Durchführung an der der Rückseite zugewandten Seite des Austragskolbens angeordnet ist und die Durchführung verschließbar ist.

Hierdurch wird verhindert, dass das Zementpulver in Richtung der Aufnahme vordringt und so den Monomerflüssigkeitstransfer behindert.

Es kann ferner vorgesehen sein, dass die Aufnahme auf der Rückseite durch den Förderkolben flüssigkeitsdicht geschlossen oder verschließbar ist, vorzugsweise flüssigkeitsdicht und gasdicht geschlossen oder verschließbar ist.

Damit wird verhindert, dass die Monomerflüssigkeit nach hinten aus der Aufnahme ausdringt. Dadurch kann das gewünschte Mischungsverhältnis sichergestellt werden. Zudem kann so eine Kontamination der Umgebung mit der Monomerflüssigkeit verhindert werden.

Gemäß einer bevorzugten Ausführung der vorliegenden Erfindung kann vorgesehen sein, dass die Kartusche hohlzylinderförmig ist und das zweite Befestigungsmittel zur Verbindung mit einer Auspressvorrichtung vorgesehen ist, wobei ein Kartuschenkopf die Vorderseite der hohlzylinderförmigen Kartusche abschließt, wobei eine Auslassöffnung im Kartuschenkopf angeordnet ist, und wobei die Auslassöffnung die Außenseite des Kartuschenkopfs mit der Innenseite des Kartuschenkopfs gasdurchlässig verbindet, wobei ein Austragsrohr am Kartuschenkopf an der Auslassöffnung angeschlossen ist, wobei eine durchstechbare Membran vorgesehen ist, welche die Rückseite des Austragskolbens flüssigkeitsundurchlässig verschließt, und die Aufnahme hohlzylinderförmig ist, wobei in einem Verbindungskolben in der Aufnahme eine für Gase und Flüssigkeiten durchlässige poröse Scheibe vorgesehen ist und mindestens eine an der Vorderseite eines Verbindungskolbens angeordnete Hohlnadel, die über einen Kanal mit der Rückseite der porösen Scheibe flüssigkeitsdurchlässig verbunden ist.

Durch diesen Aufbau wird die Lager- und Mischvorrichtung vereinfacht und besonders kostengünstig gestaltet.

Es kann vorgesehen sein, dass der Monomerflüssigkeitsbehälter durch Druckbeanspruchung zu öffnen ist. Damit kann der Monomerflüssigkeitsbehälter durch Anwendung einer linear wirkenden Kraft, wie der durch die Auspressvorrichtung, geöffnet werden.

Bevorzugten Lager- und Mischvorrichtungen können sich dadurch auszeichnen, dass der Innendurchmesser der Kartusche kleiner oder gleich dem Innendurchmesser der Aufnahme ist, vorzugsweise der Innendurchmesser der Kartusche kleiner als der Innendurchmesser der Aufnahme ist.

Hierdurch kann die Kraft, die notwendig ist, um den zähen Knochenzementteig aus der Kartusche auszupressen, durch den längeren Hub reduziert werden, ohne dass ein unnötig großer Hub zum Öffnen des Monomerflüssigkeitsbehälters und zum Auspressen der Monomerflüssigkeit aus der Aufnahme notwendig wäre.

Gemäß einer Weiterbildung kann auch vorgesehen sein, dass das Volumen der Monomerflüssigkeit im Monomerflüssigkeitsbehälter mindestens so groß ist wie das Volumen der mit Luft gefüllten Zwischenräume zwischen den Zementpulverpartikeln in der Kartusche, bevorzugt mindestens so groß ist wie das Volumen der Flüssigkeitsleitungen zwischen dem Innenraum der Kartusche und dem Innenraum der Aufnahme plus das Volumen der mit Luft gefüllten Zwischenräume zwischen den Zementpulverpartikeln in der Kartusche.

Hierdurch kann sichergestellt werden, dass das gesamte Zementpulver von der Monomerflüssigkeit benetzt werden kann und so ein homogener Knochenzementteig erzeugt wird.

Bevorzugt kann dabei vorgesehen sein, dass Zwischenräume zwischen den Zementpulverpartikeln in der Kartusche zwischen 22 Volumenprozent und 40 Volumenprozent der Pulverschüttung des Zementpulvers ausmachen.

Zur Vereinfachung der Erfüllung der hygienischen Anforderungen kann vorgesehen sein, dass In der Wandung der Aufnahme zumindest eine Belüftungsöffnung angeordnet ist, die den Innenraum der Aufnahme, in dem der Monomerflüssigkeitsbehälter angeordnet ist, mit der Umgebung verbindet, wobei bevorzugt die zumindest eine Belüftungsöffnung derart dicht im Bereich des Förderkolbens angeordnet ist, dass sie durch eine Bewegung des Förderkolbens in Richtung der Vorderseite der Aufnahme verschlossen wird, bevor der Monomerflüssigkeitsbehälter durch die Bewegung des Förderkolbens geöffnet ist.

Hierdurch kann der Innenraum der Aufnahme mit einem sterilisierenden Gas sterilisiert werden. Gleichzeitig kann die Monomerflüssigkeit nicht aus dem Innenraum der Aufnahme austreten, wenn die zumindest eine Belüftungsöffnung von dem sich in Richtung der Vorderseite der Aufnahme bewegenden Förderkolben verschlossen wird, bevor der Monomerflüssigkeitsbehälter durch die Bewegung des Förderkolbens geöffnet wird, also beispielsweise von dem Förderkolben im Innenraum der Aufnahme zerdrückt, zersplittert oder aufgerissen wird.

Vorzugsweise kann auch vorgesehen sein, dass die Kartusche eine Druckfestigkeit größer 10 bar hat, bevorzugt größer 50 bar, besonders bevorzugt größer 70 bar hat.

Hierdurch wird erreicht, dass die großen Kräfte, die beim Auspressen des zähen Knochenzementteigs aus der Kartusche auftreten, die Wandungen der Kartusche nicht so weit verformen, dass diese undicht wird oder die Bewegung des Austragskolbens blockiert.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Lager- und Mischvorrichtung hergestellt wird, wobei die Lager- und Mischvorrichtung eine Aufnahme enthaltend einen Monomerflüssigkeitsbehälter mit der Monomerflüssigkeit darin und eine Kartusche enthaltend das Zementpulver umfasst, wobei die Aufnahme an der Rückseite der Kartusche befestigt ist oder befestigt wird, wobei an der Rückseite der Kartusche eine verschließbare Durchführung vorgesehen ist, gekennzeichnet durch die folgenden nacheinander ablaufenden Schritte:
a) Einsetzen der Lager- und Mischvorrichtung in eine Auspressvorrichtung, die Auspressvorrichtung aufweisend einen axial vortreibbaren Stößel,
b) ein Förderkolben, der innerhalb der Aufnahme an deren Rückseite beweglich gelagert ist, wird mit dem Stößel in Richtung der Kartusche vorgetrieben, wobei durch die Bewegung des Förderkolbens der Monomerflüssigkeitsbehälter geöffnet wird und die Monomerflüssigkeit aus dem Monomerflüssigkeitsbehälter durch die verschließbare Durchführung in die Kartusche gepresst wird, wobei im Innenraum der Kartusche sich das Zementpulver mit der Monomerflüssigkeit mischt,
c) die Aufnahme wird von der Kartusche getrennt, wobei die verschließbare Durchführung verschlossen wird oder sich beim Trennen selbsttätig verschließt und wobei die Kartusche in der Auspressvorrichtung verbleibt oder nach der Trennung in die Auspressvorrichtung eingesetzt wird,
d) ein Austragskolben, der innerhalb der Kartusche an deren Rückseite beweglich gelagert ist, wird mit dem Stößel in Richtung der Vorderseite der Kartusche vorgetrieben, wobei durch die Bewegung des Förderkolbens die Mischung aus dem Zementpulver und der Monomerflüssigkeit aus der Kartusche als Knochenzementteig ausgetrieben wird.

Dabei kann vorgesehen sein, dass das Verfahren mit einer erfindungsgemäßen Lager- und Mischvorrichtung umgesetzt wird.

Es ist vorgesehen, dass an der Rückseite der Kartusche eine verschließbare Durchführung vorgesehen ist, durch die in Schritt b) die Monomerflüssigkeit ins Innere der Kartusche gepresst wird, wobei beim Trennen der Aufnahme von der Kartusche in Schritt c) die verschließbare Durchführung verschlossen wird oder sich beim Trennen selbsttätig verschließt.

Durch die Verschließbarkeit der Durchführung kann erreicht werden, dass nachdem die Monomerflüssigkeit in die Kartusche eingetragen wurde, durch diese keine Monomerflüssigkeit nach dem Lösen der Aufnahme von der Kartusche an der Rückseite der Kartusche austreten kann. Hierdurch wird eine Kontamination beziehungsweise Verschmutzung des OP-Bereichs mit der Monomerflüssigkeit verhindert.

Bevorzugt kann auch vorgesehen sein, dass an der Rückseite der Aufnahme ein erstes Befestigungsmittel vorgesehen ist und an der Rückseite der Kartusche ein zweites Befestigungsmittel vorgesehen ist, wobei beim Einsetzen der Lager- und Mischvorrichtung in Schritt a) die Lager- und Mischvorrichtung über das zweite Befestigungsmittel an einer Halterung der Auspressvorrichtung befestigt wird und in Schritt c) die Kartusche mit dem ersten Befestigungsmittel an der Halterung der Auspressvorrichtung befestigt wird.

Dadurch können sowohl die Aufnahme als auch die Kartusche an der gleichen Halterung einer Auspressvorrichtung, wie einer Kartuschenpistole, befestigt werden und damit die Aufnahme und die Kartusche mit der gleichen Auspressvorrichtung ausgepresst werden. Unter dem Begriff Kartuschenpistole werden auch Kartuschenpressen verstanden. Vorzugsweise wird eine manuell angetriebene Auspressvorrichtung oder Kartuschenpistole verwendet. Es kann also bevorzugt vorgesehen sein, dass das erste Befestigungsmittel und das zweite Befestigungsmittel zur Befestigung an einer Halterung einer Auspressvorrichtung geeignet und vorgesehen sind.

Zur Vereinfachung der Umsetzbarkeit des Verfahrens kann auch vorgesehen sein, dass in Schritt c) die Aufnahme von der Kartusche getrennt wird, indem eine Schraubverbindung, eine Steckverbindung, eine Rastverbindung oder ein Bajonettverschluss gelöst wird.

Hierdurch kann wird eine stabile Verbindung zwischen der Aufnahme und der Kartusche erreicht, wenn beide miteinander verbunden beziehungsweise aneinander befestigt sind.

Bevorzugt kann ferner vorgesehen sein, dass an der Vorderseite der Aufnahme ein Verbindungskolben angeordnet ist, wobei der Monomerflüssigkeitsbehälter zwischen dem Verbindungskolben und dem Förderkolben angeordnet ist, wobei in Schritt b) beim Vortreiben des Förderkolbens vor dem Einpressen der Monomerflüssigkeit in die Kartusche der Verbindungskolben in Richtung der Kartusche getrieben wird, wobei dabei ein Durchgang in die Kartusche geöffnet wird, so dass der Innenraum der Kartusche und der Innenraum der Aufnahme zwischen dem Förderkolben und dem Verbindungskolben flüssigkeitsdurchlässig miteinander verbunden werden, wobei vorzugsweise dabei oder danach der Monomerflüssigkeitsbehälter durch eine Bewegung des Förderkolbens gegen den Verbindungskolben geöffnet wird, insbesondere eine Kunststoff- oder Glasampulle als Monomerflüssigkeitsbehälter zwischen dem Förderkolben und dem Verbindungskolben zerdrückt wird, so dass die Monomerflüssigkeit im Innenraum der Aufnahme zum Einpressen in die Kartusche verfügbar ist.

Hiermit wird erreicht, dass die Flüssigkeitsverbindung zwischen dem Innenraum der Kartusche und dem Innenraum der Aufnahme erst durch eine Bewegung des Verbindungskolbens geöffnet wird. Dadurch kann eine ungewollte Öffnung des Innenraums der Kartusche vermieden werden. Des Weiteren kann durch die Bewegung des Verbindungskolbens der verschließbare Durchgang geöffnet werden, so dass die Öffnung des Verschlusses des verschließbaren Durchgangs durch den gleichen linearen Antrieb erfolgen kann, der auch zum Antreiben des Förderkolbens und des Austragskolbens verwendet wird.

Vorzugsweise werden dabei der Innenraum der Kartusche und der Innenraum der Aufnahme zwischen dem Förderkolben und dem Verbindungskolben flüssigkeitsdurchlässig und für das Zementpulver undurchlässig miteinander verbunden. Ebenfalls bevorzugt wird der Monomerflüssigkeitsbehälter zwischen dem Verbindungskolben und dem Förderkolben zusammengepresst oder in kleine Bruchstücke zerdrückt.

Gemäß einer Weiterbildung des erfindungsgemäß0en Verfahrens kann auch vorgesehen sein, dass in Schritt d) die Mischung aus dem Zementpulver und der Monomerflüssigkeit aus der Kartusche und durch eine verlängerte Austragsöffnung, ein Austragsrohr, einen Schlauch, einen Trokar und/oder einen statischen Mischer gepresst wird, wobei dort oder zuvor in der Kartusche der Knochenzementteig erzeugt wird.

Das Verfahren lässt sich besonders gut für die Vertebroplastie einsetzen. Durch den linearen zweiteiligen Aufbau und durch die Möglichkeit, die Kartusche sehr schmal zu gestalten, kann trotz des sehr zähen Knochenzementteigs und einer langen Austragsöffnung, gegebenenfalls mit Trokar und Schlauch, durch die der Fließwiderstand weiter erhöht wird, ein manuell angetriebenes Verfahren mit einer manuell angetriebenen Auspressvorrichtung verwendet werden. Die Hubstrecke, mit dem die Lager- und Mischvorrichtung angetrieben wird, ist nämlich sehr lang und durch den gestreckten Aufbau und den damit einhergehenden relativ geringen Innendurchmesser des Innenraums der Kartusche wird durch einen langen Hub nur eine geringe Menge des Knochenzementteigs durch die verlängerte Austragsöffnung gepresst.

Des Weiteren kann vorgesehen sein, dass ein von außen bedienbares Dreiwege-Ventil in der verlängerten Austragsöffnung oder hinter der Kartusche angeordnet ist, wobei das Dreiwege-Ventil in eine erste Stellung gebracht wird oder sich in der ersten Stellung befindet, wobei das Dreiwege-Ventil in der ersten Stellung eine durchgängige Verbindung von dem Innenraum der Kartusche durch die verlängerte Austragsöffnung bereitstellt, und es erfolgt in Schritt d) ein Auspressen der Kartusche mit Hilfe der Auspressvorrichtung, wobei der aus dem Zementpulver und der Monomerflüssigkeit gemischte Knochenzementteig durch das Dreiwege-Ventil und durch die verlängerte Austragsöffnung gedrückt wird, und ein nachfolgender Schritt e) erfolgt, in dem das Dreiwege-Ventil in ein zweite Stellung überführt wird, wobei das Dreiwege-Ventil in der zweiten Stellung den Fluss aus der Kartusche durch das Dreiwege-Ventil stoppt und ein Teil des hinter dem Dreiwege-Ventil in der verlängerten Austragsöffnung unter Druck stehenden Knochenzementteigs durch das Dreiwege-Ventil in einen Auffangbehälter gedrückt wird.

Ferner kann vorgesehen sein, dass nach Schritt e) in einem Schritt f) das Dreiwege-Ventil wieder in die erste Stellung überführt wird und dadurch der Knochenzementteig wieder durch das Dreiwege-Ventil durch die verlängerte Austragsöffnung geleitet wird, wobei bevorzugt anschließend die Schritte d), e) und f) chronologisch einmal oder mehrmals wiederholt werden.

Der Erfindung liegt die überraschende Erkenntnis zugrunde, dass es durch die Zweiteilung der Lager- und Mischvorrichtung und die beiden gleichen Befestigungsmittel zum Anschließen der Auspressvorrichtung gelingt, eine herkömmliche Auspressvorrichtung einzusetzen, wobei mit dem ersten Hub der Auspressvorrichtung zunächst der Monomerflüssigkeitsbehälter geöffnet wird und die Monomerflüssigkeit durch eine Verbindung der beiden Teile, nämlich der Aufnahme und der Kartusche, in das Zementpulver gepresst wird und anschließend ein Teil, nämlich die Aufnahme, vom anderen Teil der Lager- und Mischvorrichtung getrennt wird und die Auspressvorrichtung anschließend mit dem zweiten Hub den Knochenzementteig aus der Kartusche austrägt. Hierdurch kann auch eine besonders dünne Kartusche mit geringem Innendurchmesser verwendet werden, so dass der zähe und hochviskose Knochenzementteig auch gegen den Widerstand eines Schlauchs noch ausgepresst und appliziert werden kann, ohne dass die Auspressvorrichtung hierfür sehr langgestreckt mit einem besonders langen Stößel aufgebaut werden müsste. Dadurch sind die Lager- und Mischvorrichtung und das Verfahren auch für die Vertebroplastie einsetzbar.

Dem erfindungsgemäßen Verfahren und der erfindungsgemäßen Lager- und Mischvorrichtung liegt die gemeinsame Idee zugrunde, dass eine zunächst verbundene zweiteilige Lager- und Mischvorrichtung genutzt wird, um mit einer Auspressvorrichtung den Monomerflüssigkeitsbehälter zu öffnen und die Monomerflüssigkeit in die Kartusche zu transferieren, anschließend die Kartusche von der restlichen Lager- und Mischvorrichtung getrennt wird und die mit der selben Auspressvorrichtung der Knochenzementteig aus der Kartusche ausgetrieben werden kann. Hierzu werden bevorzugt gleichartige Befestigungsmittel an der Kartusche und an der Aufnahme eingesetzt, die mit dem selben Gegenbefestigungsmittel an der Auspressvorrichtung verbunden werden. Durch geeigneten Aufbau der Lager- und Mischvorrichtung beziehungsweise durch geeignete Verfahrensschritte kann erreicht werden, dass eine manuell angetriebene Auspressvorrichtung einsetzbar ist. Ferner wird durch geeignete erfindungsgemäße Maßnahmen erreicht, dass beim Lagern und Mischen des Knochenzementteigs keine Monomerflüssigkeit und kein Knochenzementteig aus der Lager- und Mischvorrichtung austritt und die Umgebung kontaminiert. Schließlich kann durch die erfindungsgemäßen Maßnahmen die Lager- und Mischvorrichtung als hygienisches nur einmal verwendbares Wegwerfprodukt aufgebaut werden.

Der besondere Vorteil der erfindungsgemäßen Lager- und Mischvorrichtung besteht darin, dass mit konventionellen, manuell angetriebenen Auspressvorrichtungen, die für normale PMMA-Zemente üblich sind, ein Zweikomponenten-Spinezement beziehungsweise der Knochenzementteig für die Vertebroplastie über einen dünnen Schlauch in einen Trokar ausgepresst werden kann. Die Augmentation von Wirbelkörpern erfolgt unter permanenter Röntgenkontrolle. Der Schlauch zwischen dem Trokar und dem Applikator ermöglicht es, dass der Arzt mit seinen Händen nicht im Bereich der Röntgenstrahlung arbeitet. Es sind dabei keine komplexen, teuren Hydraulik-Applikationsvorrichtungen notwendig.

Ferner wurde überraschend gefunden, dass es durch die Verschließbarkeit der Verbindung zwischen den beiden Teilen an der Rückseite der Kartusche möglich ist, die Kartusche nach dem Eintrag der Monomerflüssigkeit in das Zementpulver von der restlichen Lager- und Mischvorrichtung zu trennen, ohne dass verbliebene Monomerflüssigkeit an der Rückseite der Kartusche austritt. Gleichzeitig ist das System schnell einsatzbereit und ohne großen Aufwand zu bedienen. Die Lager- und Mischvorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Im Folgenden werden weitere Ausführungsbeispiele der Erfindung anhand von vierzehn schematisch dargestellten Figuren erläutert, ohne jedoch dabei die Erfindung zu beschränken. Dabei zeigt:
Figur 1: eine schematische Querschnittansicht einer beispielhaften erfindungsgemäßen Lager- und Mischvorrichtung;
Figur 2: eine schematische Seitenansicht der Lager- und Mischvorrichtung nach Figur 1;
Figur 3: vier schematische Querschnittansichten der Lager- und Mischvorrichtung nach den Figuren 1 und 2 übereinander zur Verdeutlichung des Ablaufs eines erfindungsgemäßen Verfahrens;
Figur 4: eine schematische Querschnittansicht durch den vorderen Teil der erfindungsgemäßen Lager- und Mischvorrichtung nach den Figuren 1 bis 3 mit einer angeschlossenen verlängerten Austragsöffnung;
Figur 5: drei schematische perspektivische Ansichten auf Teile erfindungsgemäßer Lager- und Mischvorrichtungen nach den Figuren 1 bis 4;
Figur 6: eine schematische Querschnittansicht als Detail-Ansicht eines Dreiwege-Ventils für eine verlängerte Austragsöffnung nach Figur 4;
Figur 7: eine schematische Querschnittansicht als Detail-Ansicht des Dreiwege-Ventils nach Figur 6 mit einer Schnittebene senkrecht zur Flussrichtung des Knochenzementteigs;
Figur 8: eine schematische Querschnittansicht als Detail-Ansicht der Verbindung der beiden Teile der Lager- und Mischvorrichtung während des Einsatzes nach der 2. Abbildung von oben in Figur 3;
Figur 9: eine schematische Querschnittansicht als Detail-Ansicht der Verbindung der beiden Teile der Lager- und Mischvorrichtung vor dem Austrag nach der 4. Abbildung von oben in Figur 3;
Figur 10: eine schematische perspektivische Teilansicht (oben) und eine schematische Teil-Querschnittansicht (unten) als Detail-Ansicht der Rückseite des hinteren Teils einer Lager- und Mischvorrichtung im Ausgangszustand;
Figur 11: zwei schematische perspektivische Querschnittansichten durch ein Ventilsystem für eine alternative erfindungsgemäße Lager- und Mischvorrichtung und zwar ein Dreiwege-Ventil in geschlossener Position beziehungsweise Stellung (Figur 11 oben) und in geöffneter Position beziehungsweise Stellung (Figur 11 unten);
Figur 12: zwei schematische Querschnittansichten in Aufsicht durch das Ventilsystem nach Figur 11 und zwar das Dreiwege-Ventil in geöffneter Position beziehungsweise Stellung (Figur 12 unten) und in geschlossener Position beziehungsweise Stellung (Figur 12 oben);
Figur 13: eine schematische Querschnittansicht durch ein drittes erfindungsgemäßes Ausführungsbeispiel für eine Lager- und Mischvorrichtung; und
Figur 14: eine Ausschnittvergrößerung der Verbindung der beiden Teile des dritten Ausführungsbeispiels nach Figur 13 vor Beginn des Auspressvorgangs als Detailansicht.

In den Figuren werden der Einfachheit halber teilweise bei gleichen Bauteilen die gleichen Bezugszeichen auch für unterschiedliche Ausführungsformen verwendet.

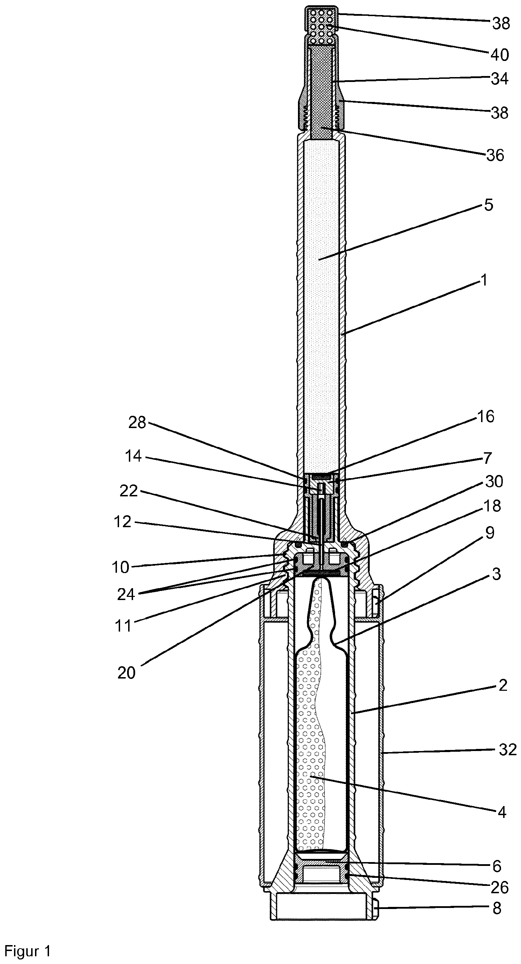

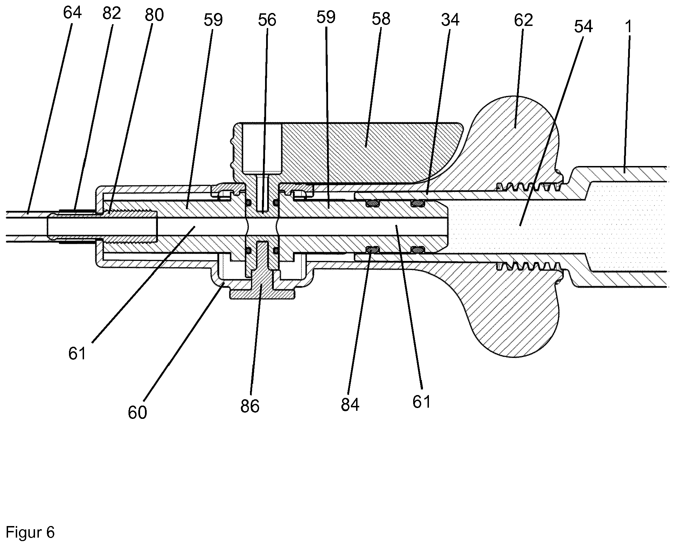

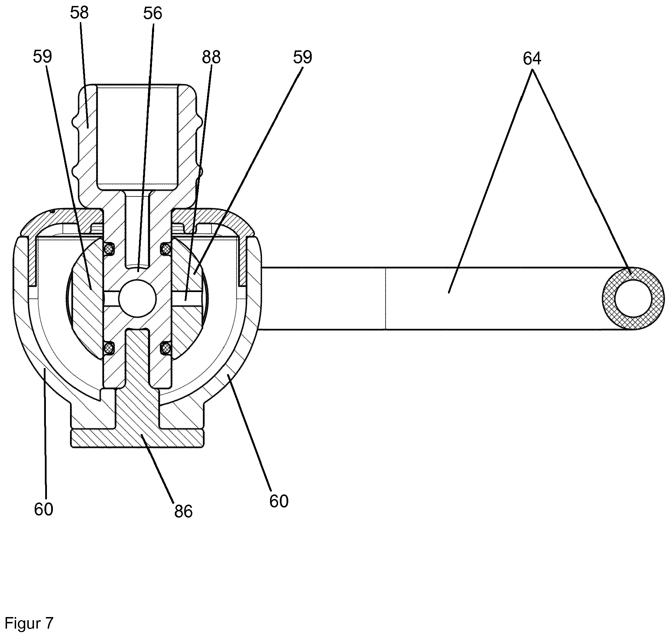

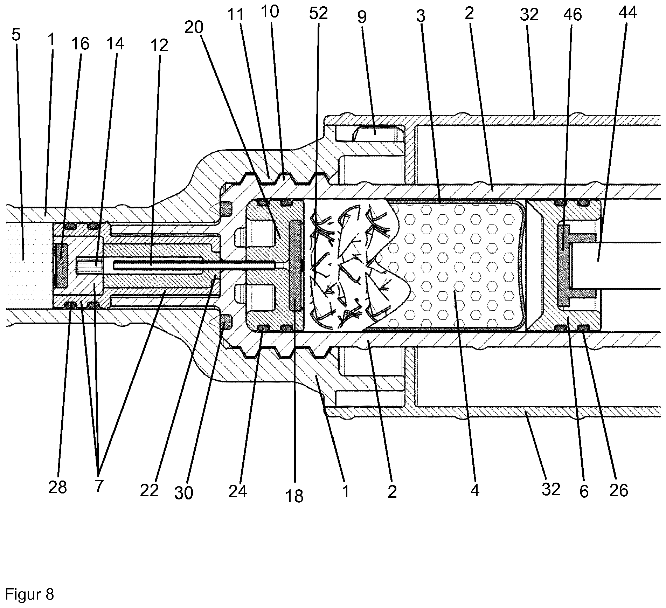

Die Figuren 1 bis 5 zeigen verschiedene schematische Ansichten einer ersten beispielhaften erfindungsgemäßen Lager- und Mischvorrichtung. Die Figuren 6 und 7 zeigen schematische Querschnittansichten als Detailansichten durch ein Ventilsystem, das als Teil der erfindungsgemäßen Lager- und Mischvorrichtung nach den Figuren 1 bis 5 verwendet werden kann. Die Figuren 8 bis 10 zeigen weitere Detailansichten der erfindungsgemäßen Lager- und Mischvorrichtung nach den Figuren 1 bis 5.

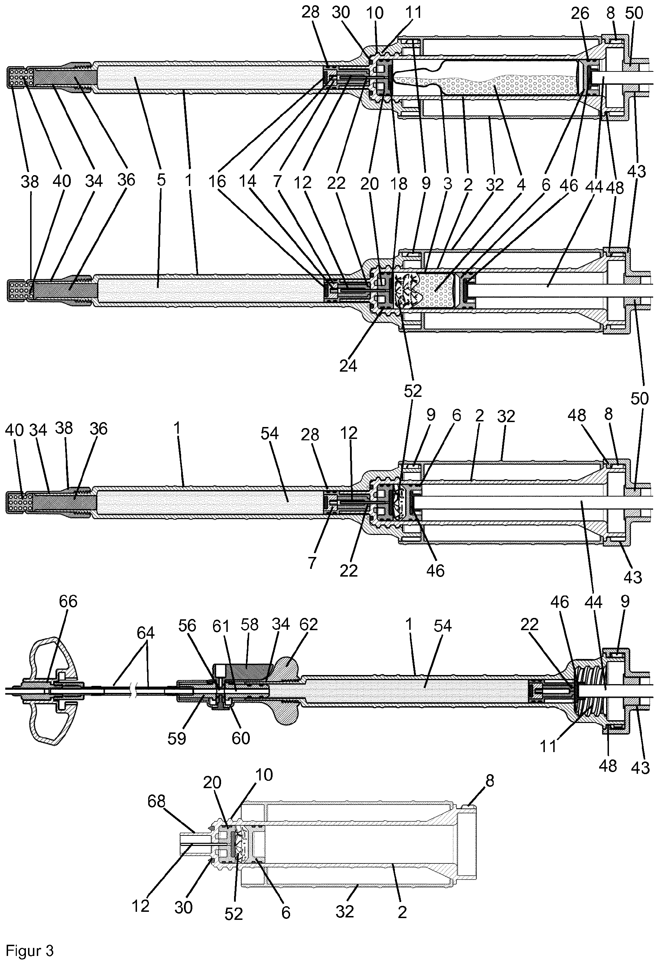

Die Lager- und Mischvorrichtung hat als vorderen Teil (in den Figuren 1, 2 und 4 oben, in den Figuren 3, 5, 6, 8 und 9 links) eine Kartusche 1 aus Kunststoff mit einem zylindrischen Innenraum. Die Kartusche 1 ist mit einer Aufnahme 2 aus Kunststoff für eine Glasampulle 3 (oder Kunststoffampulle 3) lösbar verbunden. Die Aufnahme 2 weist ebenfalls einen zylindrischen Innenraum auf, in den die Glasampulle 3 eingesteckt ist. In der Glasampulle 3 befindet sich die Monomerflüssigkeit 4. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten 4, 5 für einen PMMA-Knochenzement, der mit der Lager- und Mischvorrichtung hergestellt werden kann. Aufgrund der Glasampulle 3 kann die Monomerflüssigkeit 4 sehr lange in der Aufnahme 2 und dadurch in der Lager- und Mischvorrichtung gelagert werden.

In der Aufnahme 2 ist ein im zylindrischen Innenraum der Aufnahme 2 in Längsrichtung beweglicher Förderkolben 6 aus Kunststoff angeordnet. Der Förderkolben 6 ist im Bereich der Rückseite der Aufnahme 2 angeordnet. Die Glasampulle 3 kann mit dem Förderkolben 6 in der Aufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Aufnahme 2 abgestreift werden.

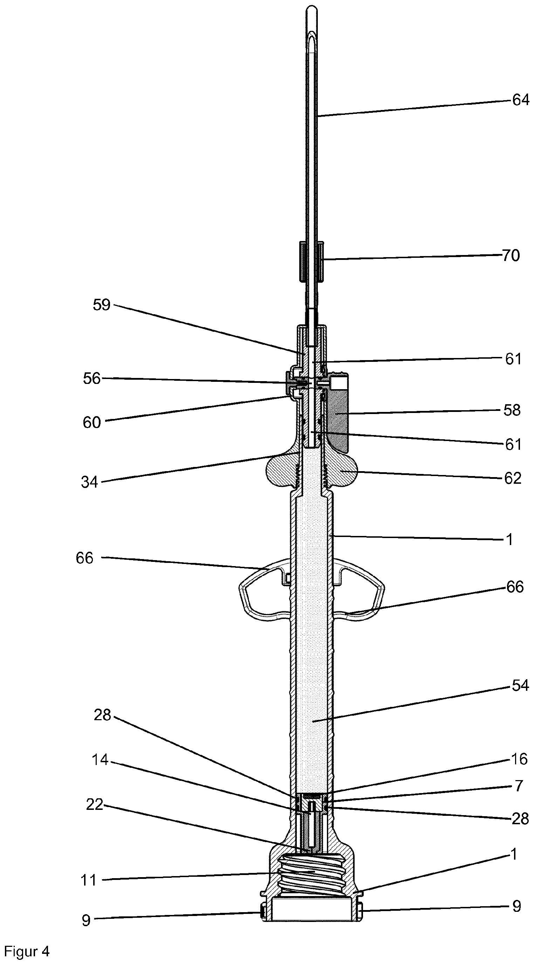

In dem Innenraum der Kartusche 1 ist in dessen Rückseite (in den Figuren 1, 2 und 4 Richtung unten, in den Figuren 3, 5, 6, 8 und 9 Richtung rechts) ein Austragskolben 7 aus Kunststoff angeordnet. An der Rückseite der Aufnahme 2 ist ein Befestigungsmittel 8 vorgesehen, mit dem die Aufnahme 2 an einer Auspressvorrichtung 43 (in Figur 1 nicht zu sehen, siehe aber Figur 3) verbunden werden kann. Ein baugleiches Befestigungsmittel 9 ist an der Rückseite der Kartusche 1 vorgesehen. Sowohl die Aufnahme 2 als auch die Kartusche 1 können also an der gleichen Auspressvorrichtung 43 befestigt werden. Die Befestigungsmittel 8, 9 sind vorzugsweise zur Bildung eines Bajonettverschlusses 8, 9 geeignet und vorgesehen. Dadurch kann der Förderkolben 6, der von der Rückseite der Aufnahme 2 aus frei zugänglich ist, mit der selben Auspressvorrichtung 43 in Richtung der Vorderseite vorgetrieben werden, wie der Austragskolben 7, der von der Rückseite der Kartusche 1 aus frei zugänglich ist, wenn die Kartusche 1 nicht mit der Aufnahme 2 verbunden ist (siehe beispielsweise Figur 4).

Die Kartusche 1 und die Aufnahme 2 sind über ein Außengewinde 10 an der Vorderseite der Aufnahme 2 und ein Innengewinde 11 an der Rückseite der Kartusche 1 lösbar miteinander verbunden beziehungsweise lösbar miteinander verbindbar. Die Aufnahme 2 und die Kartusche 1 sind für die Monomerflüssigkeit 4 flüssigkeitsdurchlässig miteinander über ein Röhrchen 12 aus einem Metall oder aus Kunststoff und eine Durchführung 14 im Austragskolben 7 verbunden. Die Durchführung 14 durch den Austragskolben 7 mündet durch einen für das Zementpulver 5 undurchlässigen aber für die Monomerflüssigkeit 4 durchlässigen Porenfilter 16 in den Innenraum der Kartusche 1. Vor dem Eintritt in den Innenraum der Kartusche 1 verzweigt die Durchführung 14 in mehrere Arme, wobei die Arme in dem Schnitt nach Figur 1 nicht zu sehen sind, da sie nicht in der gezeigten und geschnitten dargestellten Mittelebene der Lager- und Mischvorrichtung liegen.

In der Verbindung zum Röhrchen 12 ist in der Aufnahme 2 ein Filter 18 angeordnet, mit dem die Splitter der Glasampulle 3 zurückgehalten werden können. Statt dem Filter 18 oder zusätzlich zum Filter 18 kann auch ein Sieb vorgesehen sein. Der Filter 18 ist in einem Verbindungskolben 20 angeordnet, an dem auch das Röhrchen 12 befestigt ist. Der Verbindungskolben 20 liegt bei dieser Ausführung an der Vorderseite der Aufnahme 2 innen an und dient lediglich der Erleichterung des Zusammenbaus der Lager- und Mischvorrichtung. Theoretisch kann das Röhrchen 12 und der Filter 18 auch fest mit der Aufnahme 2 verbunden sein und der Verbindungskolben 20 weggelassen werden.

Das Röhrchen 12 ist durch ein Ventil mit drei Dichtlippen 22 in die Durchführung 14 durch den Austragskolben 7 geführt. Die drei Dichtlippen 22 sind durch einen Y-Schnitt in einem elastischen Kunststoff-Pfropfen realisiert, wobei der Kunststoff-Pfropfen den rückseitigen Teil des Austragskolbens 7 bildet. Im Ausgangszustand ist das Röhrchen 12 durch diese Dichtlippen 22 geführt und der Durchgang 14 in Richtung der Rückseite von den an dem Röhrchen 12 anliegenden Dichtlippen 22 flüssigkeitsdicht angedichtet. Wenn das Röhrchen 12 aus dem Pfropfen beziehungsweise aus den Dichtlippen 22 herausgezogen wird, dichten die Dichtlippen 22 den Austragskolben 7 und damit die Kartusche 1 rückseitig flüssigkeitsdicht ab.

Der zylindrische Verbindungskolben 20 hat einen zur Zylindergeometrie des Innenraums der Aufnahme 2 passenden Außenumfang und ist über zwei umlaufende Dichtungen 24 gegen die Innenwand der Aufnahme 2 flüssigkeitsdicht abgedichtet. Ebenso ist der Förderkolben 6 über zwei umlaufende Dichtungen 26 gegen die Innenwand der Aufnahme 2 flüssigkeitsdicht abgedichtet und der Austragskolben 7 ist über zwei umlaufende Dichtungen 28 gegen die Innenwand der Kartusche 1 flüssigkeitsdicht abgedichtet. Des Weiteren sind die Kartusche 1 und die Aufnahme 2 über eine an der Frontfläche der Aufnahme 2 befestigte Dichtung 30 gegeneinander abgedichtet, wenn die Kartusche 1 und die Aufnahme 2 aneinander befestigt sind. Alle diese Dichtungen 24, 26, 28, 30 dienen dazu, einen Austritt von Monomerflüssigkeit 4 oder von Knochenzement zu verhindern, um eine Kontamination der Umgebung (des OP-Saals und des Anwenders) zu verhindern. Die Dichtungen 24, 26, 28, 30 können hierzu aus Gummi bestehen.

Die Aufnahme 2 ist zusätzlich von einem Gehäuse 32 aus einem Kunststoff, wie Plastik, umgeben, wobei das Gehäuse 32 mit der Aufnahme 2 verbunden ist und an der Vorderseite die Befestigungsmittel 9 der Kartusche 1 abdeckt, wenn die Kartusche 1 mit der Aufnahme 2 verbunden ist. Die Kartusche 1, die Aufnahme 2 und das Gehäuse 32 können durch Spritzgießen hergestellt werden.

Die Vorderseite der Kartusche 1 mündet in ein Austragsrohr 34, das ein Außengewinde aufweist. Im Inneren der Austragsrohrs 34 ist ein Porenfilter 36 angeordnet, der für das Zementpulver 5 undurchlässig aber für Gase durchlässig ist. An dem Außengewinde des Austragsrohrs 34 ist eine Kappe 38 befestigt, wobei der vordere Teil der Kappe 38 mit einem Styropor oder Schaumstoff 40 gefüllt ist. An der Kappe 38 sind zwei Flügel 42 vorgesehen, so dass die Kappe 38 nach Art einer Flügelschraube bequem von dem Austragsrohr 34 abgeschraubt werden kann. Die Kappe 38 weist seitliche Öffnungen auf. Durch diesen Aufbau kann das Innere der Kartusche 1 und das Zementpulver 5 mit Hilfe von Ethylenoxid sterilisiert werden, da die Öffnungen in der Kappe 38, das Styropor oder der Schaumstoff 40, der Porenfilter 36 und die Zwischenräume zwischen den Pulverpartikeln des Zementpulvers 5 luftdurchlässig sind. Gleichzeitig kann Luft aus der Aufnahme durch das Zementpulver 5, den Porenfilter 36, das Styropor oder der Schaumstoff 40 und die Öffnungen in der Kappe 38 herausgepresst werden, wenn der Förderkolben 6 in Richtung der Aufnahme 1 gepresst wird.

Das Zementpulver 5 ist in der Kartusche 1 eingeschlossen, da alle Öffnungen mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Lager- und Mischvorrichtung auch zum langfristigen Lagern des Zementpulvers 5 geeignet.

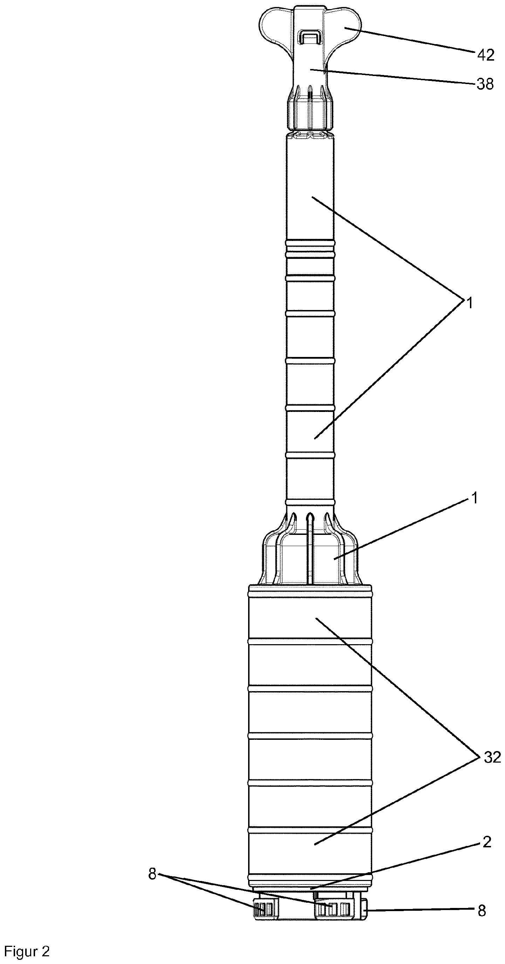

Figur 3 zeigt vier schematische Querschnittansichten der Lager- und Mischvorrichtung nach den Figuren 1 und 2 übereinander zur Verdeutlichung des Ablaufs eines erfindungsgemäßen Verfahrens. Zu Beginn des Verfahrens liegt die Lager- und Mischvorrichtung im Ausgangszustand vor, so wie sie auch in Figur 1 gezeigt ist. Die Kartusche 1 und die Aufnahme 2 der Lager- und Mischvorrichtung sind also zunächst miteinander über die Gewinde 10, 11 miteinander verbunden. In diesem Zustand wird die Lager- und Mischvorrichtung in eine Auspressvorrichtung 43 in Form einer herkömmlichen Kartuschenpistole eingesetzt und mit dem Befestigungsmittel 8 an der Auspressvorrichtung 43 befestigt (siehe oberste Abbildung in Figur 3). Von der Auspressvorrichtung 43 ist nur der vordere Teil dargestellt. Die Auspressvorrichtung 43 umfasst einen linear vortreibbaren Stößel 44. Bevorzugt wird der Stößel 44 manuell angetrieben. Der Stößel 44 endet an seiner Vorderseite in einem Teller 46, mit dem auf den Förderkolben 6 gedrückt werden kann. Die Auspressvorrichtung 43 ist hierzu über ein Gegenbefestigungsmittel 48 an die Rückseite der Aufnahme 2 angeschlossen, so dass der Teller 46 beim Vortreiben des Stößels 44 auf den Förderkolben 6 drückt und diesen in Richtung der Kartusche 1 vortreibt. Hierzu ist der Stößel 44 gegen ein Lager 50 und darüber gegen das Gegenbefestigungsmittel 48 und damit gegen die Aufnahme 2 linear beweglich gelagert.

Die Auspressvorrichtung 43 wird bedient und dabei der Stößel 44 und mit dem Stößel 44 der Förderkolben 6 in Richtung der Kartusche 1 vorgetrieben. Da die Kunststoff- oder Glasampulle 3 an der Vorderseite an dem Verbindungskolben 20 anliegt, verkleinert sich der Innenraum der Aufnahme 2 und die Glasampulle 3 zerbricht und die Monomerflüssigkeit 4 tritt aus der Glasampulle 3 in den Innenraum der Aufnahme 2 aus. Diese Situation ist in Figur 3, 2. Abbildung von oben sowie in einer vergrößerten Ausschnittansicht in Figur 8 gezeigt. Überstehende Luft aus der Aufnahme 2 wird durch den Filter 18, die Durchführung 14, den Porenfilter 16, durch die Zwischenräume zwischen den Partikeln des Zementpulvers 5, durch den Porenfilter 36, durch den Schaumstoff 40 und aus den Öffnungen in der Kappe 38 aus der Lager- und Mischvorrichtung herausgedrückt.

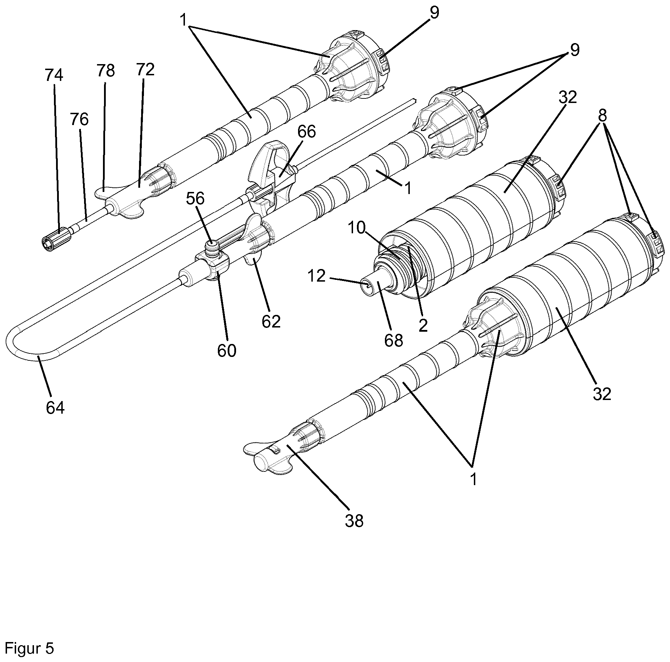

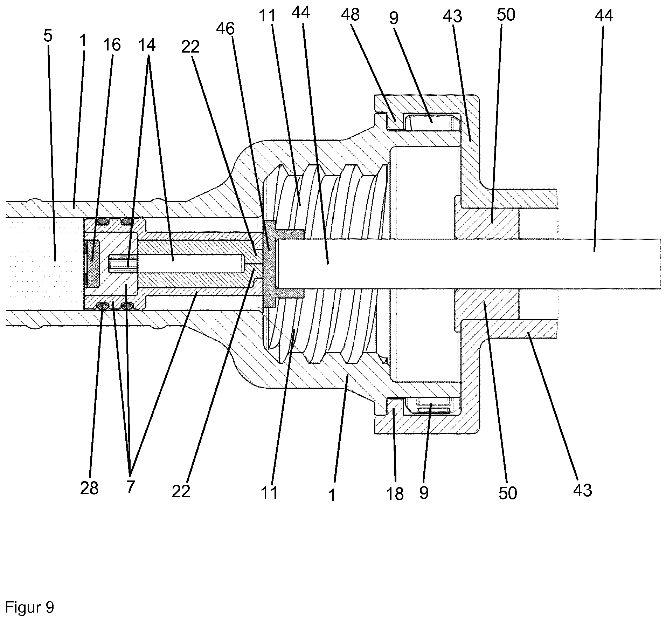

Von der Glasampulle 3 bleiben schließlich nur kleine Splitter 52 zurück, die von dem Filter 18 zurückgehalten werden und in der Aufnahme 2 verbleiben. Die Monomerflüssigkeit 4 wird durch den Filter 18, die Durchführung 14 und den Porenfilter 16 in das Zementpulver 5 gepresst und beginnt dort mit dem Zementpulver 5 zu reagieren, so dass sich aus dem Gemisch 54 der Knochenzementteig 54 bildet. Diese Situation ist in Figur 3, dritte Abbildung von oben gezeigt. Zudem ist die Kartusche 1 mit der verlängerten Austragsöffnung auch in Figur 4 und Figur 5 gezeigt. In diesem Zustand wird die Lager- und Mischvorrichtung aus der Auspressvorrichtung 43 entnommen. Die Aufnahme 2 und das Gehäuse 32 werden von der Kartusche 1 abgeschraubt. Zudem wird die Kappe 38 mit dem Porenfilter 36 und dem Schaumstoff 40 abgeschraubt und eine verlängerte Austragsöffnung auf das Austragsrohr 34 aufgeschraubt. Anschließend wird die Kartusche 1 mit der verlängerten Austragsöffnung wieder an der Auspressvorrichtung 43 befestigt. Zuvor wurde die Auspressvorrichtung 43 zurückgesetzt, das heißt, der Stößel 44 wurde wieder zurückgeschoben und in die Ausgangsstellung überführt. Nach dem Herausziehen des Röhrchens 12 verschließen sich die Dichtlippen 22 von selbst, so dass keine Monomerflüssigkeit 4, die sich noch in der Durchführung 14 befindet, an der Rückseite der Kartusche 1 austreten kann, wenn die Kartusche 1 von der Aufnahme 2 getrennt ist (siehe hierzu auch die Detailansicht der Ausschnittvergrößerung nach Figur 9).

Zur Befestigung der Auspressvorrichtung 43 an der Kartusche 1 greifen die Gegenbefestigungsmittel 48 der Auspressvorrichtung 43 in die Befestigungsmittel 9 an der Rückseite der Kartusche 1. Da die Befestigungsmittel 9 an der Kartusche 1 und die Befestigungsmittel 8 an der Aufnahme 2 gleich sind, können beide am selben Gegenbefestigungsmittel 48 der selben Auspressvorrichtung 43 befestigt werden. Dies vereinfacht den Aufbau und ermöglicht die Verwendung herkömmlicher Auspressvorrichtungen 43. Die Befestigungsmittel 8, 9 und das Gegenbefestigungsmittel 48 bilden dabei einen Bajonettverschluss.

Die verlängerte Austragsöffnung umfasst ein Dreiwege-Ventil 56, das von außen über einen Knebelgriff 58 manuell bedient werden kann. Das Dreiwege-Ventil 56 sitzt in dichter Passung in einem Rohr 59, das deinen Ventilsitz 59 bildet. Die Figuren 6 und 7 zeigen schematische Querschnittansichten als Detailansichten durch ein Ventilsystem, das als Teil der erfindungsgemäßen Lager- und Mischvorrichtung nach den Figuren 1 bis 5 verwendet werden kann. Im Bereich des Dreiwege-Ventils 56 ist ein nach außen geschlossener Auffangbehälter 60 zur Aufnahme von Knochenzementteig 54 angeordnet. In dem Rohr 59 ist ein Durchgang 61 vorgesehen, der das Innere des Rohrs 59 bildet. Der Durchgang 61 in der Vorderseite des Rohrs 59 kann über das Dreiwege-Ventil 56 mit der Aufnahme 60 verbunden werden, so dass unter Druck stehender Knochenzementteig 54 von der vorderen Seite der verlängerten Austragsöffnung nach hierhin ausweichen kann und der Knochenzementteig 54 nur wenig aus der verlängerten Austragsöffnung nachfließt, wenn das Dreiwege-Ventil 56 in dieser geschlossenen Stellung ist. Hierdurch wird die Menge an nachströmendem Knochenzementteig 54 reduziert, wenn das Dreiwege-Ventil 56 verwendet wird. Das Dreiwege-Ventil 56 wird mit Hilfe von Flügeln 62 auf das Austragsrohr 34 aufgeschraubt. Der vordere Teil des Rohrs 59 ist mit einem Schlauch 64 verbunden, der in einen Trokar 66 mündet.

Der abgeschraubte rückseitige Teil der Lager- und Mischvorrichtung, nämlich die Aufnahme 2 mit den Splittern 52 darin und das Gehäuse 32, bleibt zurück und kann entsorgt werden. An der Vorderseite der Aufnahme 2 ist ein Schutzrohr 68 vorgesehen, in dem das Röhrchen 12 angeordnet ist, so dass sich der Anwender nicht so leicht an dem Röhrchen 12 verletzen kann. Über den Schlauch 64 und den Trokar 66 kann der Knochenzementteig 54 ausgetragen werden. Dazu wird der Austragskolben 7 mit dem Stößel 44 und dem Teller 46 in Richtung des Austragsrohrs 34 vorgetrieben. Der Knochenzementteig 54 aus dem Inneren der Kartusche 1 wird bei geöffnetem Dreiwege-Ventil 56, sowie es in Figur 3 (unterste Abbildung) und in den Figuren 6 und 7 gezeigt ist, durch das Austragsrohr 34, durch das Dreiwege-Ventil 56 und den Durchgang 61, durch den Schlauch 64 und den Trokar 66 ausgetrieben und kann dort an den Wirbeln eines Patienten appliziert werden oder theoretisch zur weiteren Verarbeitung verwendet werden. Zur Unterbrechung des Flusses des Knochenzementteigs 54 kann das Dreiwege-Ventil 56 bedient werden. Unter Druck stehender Knochenzementteig 54 aus dem Schlauch 64 und dem Trokar 66 kann durch das Dreiwege-Ventil 56 in den Auffangbehälter 60 abfließen, ohne die Umgebung zu kontaminieren. Dadurch wird der Fluss des Knochenzementteigs 54 schnell unterbrochen.

Der Trokar 66 ist über ein Luer-System-Adapter 70 an den Schlauch 64 angeschlossen (siehe Figur 4 oder zweite Ausführung von oben links in Figur 5). Der Trokar 66 kann auch direkt mit dem Schlauch 64 verbunden sein. In Figur 5 ist bei der ersten Ausführung von oben links eine Variante gezeigt, bei der ein Anschluss 72 einen Luer-System-Adapter 74 über einen kurzen Schlauch 76 mit der Kartusche 1 verbindet. Der Anschluss 72 kann mit Hilfe von Flügeln 78 nach Art einer Flügelschraube auf das Austragsrohr 34 der Kartusche 1 geschraubt werden. Hierzu weist der Anschluss 72 ein passendes Innengewinde auf.

In der Detailansicht der Ausschnittvergrößerung nach Figur 6 des Ventilsystems ist zusätzlich zu sehen, dass das Rohr 59 mit einem Einsatz 80, der einen mit dem Durchgang 61 fluchtenden Kanal aufweist, mit dem Schlauch 64 verbunden ist. Dazu ist der Einsatz 80 in das Rohr 59 eingeschraubt. Um eine druckdichte Verbindung zu gewährleisten, ist der Schlauch 64 mit einer Metallhülse 82 auf den Einsatz 80 gekrimpt und das Rohr 59 mit zwei umlaufenden Dichtungen 84 gegen die Innenwand des Austragsrohrs 34 abgedichtet. Die Unterseite des Dreiwege-Ventils 56 ist mit einem Stopfen 86 abgesichert, so dass das Dreiwege-Ventil 56 nicht einfach aus dem Ventilsitz 59 beziehungsweise dem Rohr 59 herausgezogen werden kann. In der Schnittebene nach Figur 7, die zu der Schnittebene nach Figur 6 senkrecht liegt und die senkrecht zum Durchgang 61 liegt, ist ein Ablasskanal 88 im Ventilsitz 59 zu erkennen, durch die bei geschlossener Stellung des Dreiwege-Ventils 56 der Knochenzementteig 54 von der vorderen Seite der verlängerten Austragsöffnung in den Auffangbehälter 60 abfließen kann.

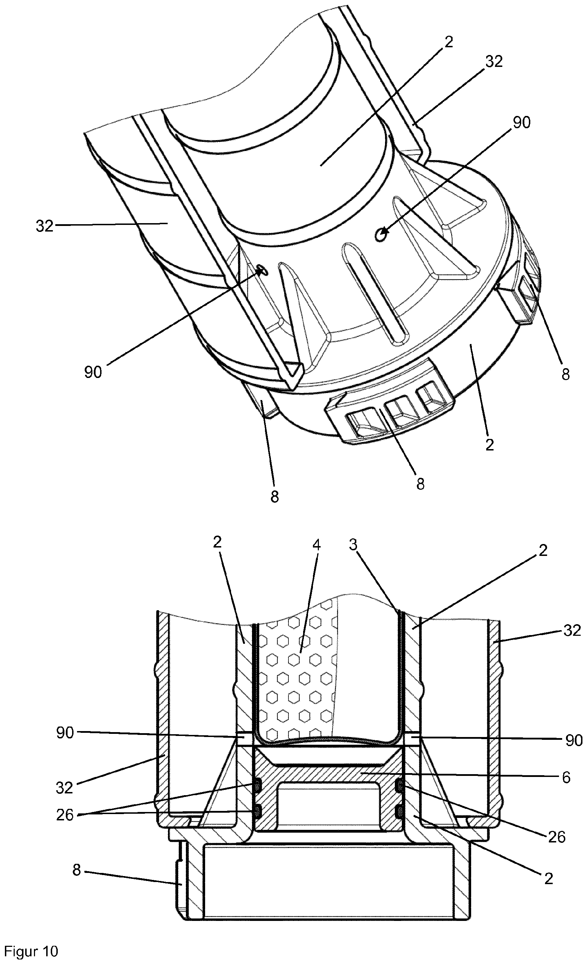

Figur 10 zeigt eine schematische perspektivische Teilansicht (oben) und eine schematische Teil-Querschnittansicht (unten) als Detail-Ansicht der Rückseite des hinteren Teils einer Lager- und Mischvorrichtung im Ausgangszustand. Dort ist zu erkennen, dass mehrere Belüftungsöffnungen 90 in der Wandung der Aufnahme 2 vorgesehen sind, durch die der Innenraum der Aufnahme 2 mit Hilfe eines sterilisierenden Gases wie Ethylenoxid sterilisiert werden kann. Die Belüftungsöffnungen 90 sind unmittelbar benachbart zum Förderkolben 6 angeordnet, so dass der Förderkolben 6 die Belüftungsöffnungen 90 unmittelbar verschließt, wenn er in Richtung der Kartusche 1 vorgetrieben wird. Dadurch wird verhindert, dass Monomerflüssigkeit 4 durch die Belüftungsöffnungen 90 austreten kann, wenn die Glasampulle 3 in der Aufnahme 2 geöffnet wurde.

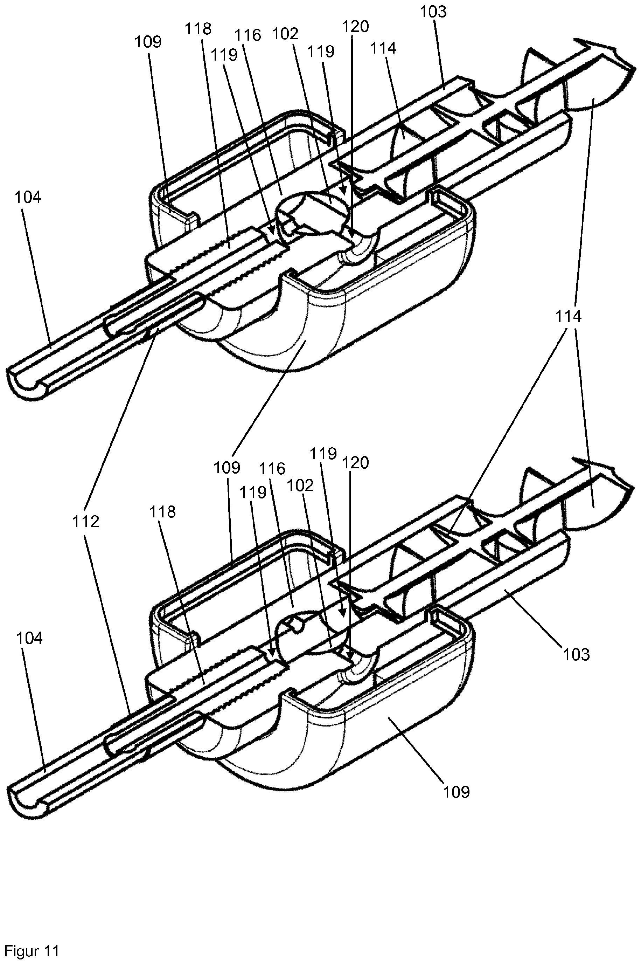

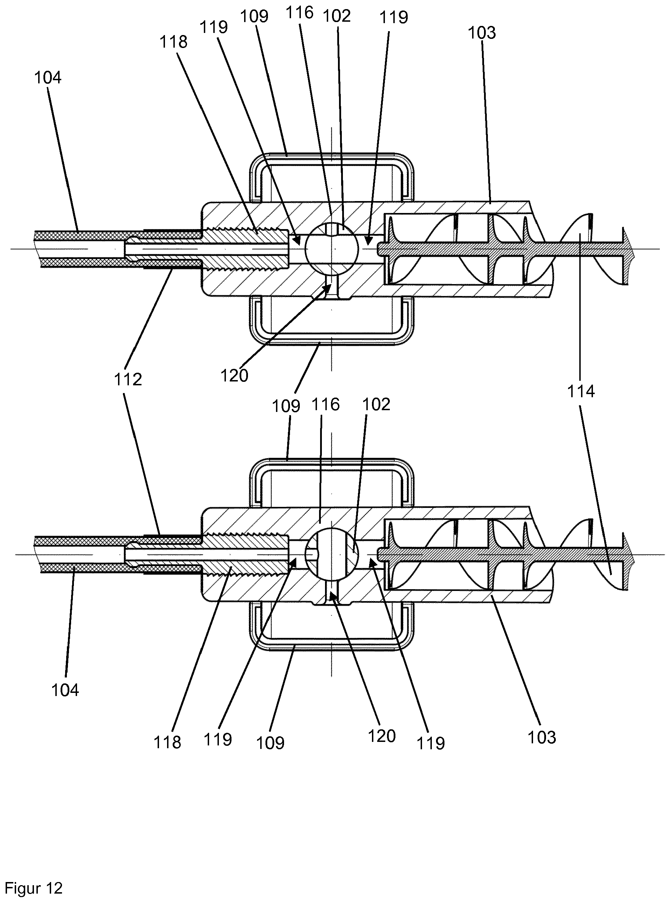

Die Figuren 11 und 12 zeigen jeweils zwei schematische perspektivische Querschnittansichten durch ein beispielhaftes Dreiwege-Ventil 102 für eine zweite alternative erfindungsgemäße Lager- und Mischvorrichtung und zwar das Dreiwege-Ventil 102 in geschlossener Position beziehungsweise Stellung (Figur 11 oben und Figur 12 unten) und in geöffneter Position beziehungsweise Stellung (Figur 11 unten und Figur 12 oben), um die Funktionsweise des Dreiwege-Ventils 102 anhand des inneren Aufbaus zu erläutern.

Der Aufbau der alternativen zweiten erfindungsgemäßen Lager- und Mischvorrichtung gleicht dort, wo es nicht anders beschrieben oder in den Figuren 11 und 12 zu sehen ist, dem vorherigen ersten Ausführungsbeispiel nach den Figuren 1 bis 10.

Ein Rohr 103 ist als verlängerte Austragsöffnung an der Vorderseite einer Kartusche (in den Figuren 11 und 12 nicht zu sehen, aber wie nach dem vorherigen Ausführungsbeispiel ausgeführt) angeordnet. Hinter dem Ventilsystem ist ein Schlauch 104 an dem Ventilsystem befestigt, durch den der Knochenzementteig (in den Figuren 11 und 12 nicht dargestellt) ausgetragen werden kann. Damit der Knochenzementteig nicht unkontrolliert austreten kann ist analog der Ausführung nach den Figuren 6 und 7 ein Auffangbehälter 109 vorgesehen, der zur Aufnahme von aus dem Dreiwege-Ventil 102 austretendem Knochenzement vorgesehen ist. Dadurch wird eine Kontamination der Umgebung - also insbesondere des OP-Bereichs - mit Knochenzementteig verhindert. Der Schlauch 104 ist mit einer Hülse 112 aus Metall über einen Krimp-Anschluss durchdicht mit dem Ventilsystem verbunden.

Im Inneren des Rohrs 103 befindet sich ein statischer Mischer 114, der sich bis an das Dreiwege-Ventil 102 heran erstreckt. Mit Hilfe des statischen Mischers 114 werden die Ausgangskomponenten des Knochenzements beziehungsweise der vorgemischte Knochenzementteig durchmischt, wenn diese durch den statischen Mischer 114 im Rohr 103 gepresst werden.

Das drehbare Dreiwege-Ventil 102 ist in den Querschnittansichten nach den Figuren 11 und 12 in der Symmetrieebene der darin zu erkennenden Kanäle geschnitten. Die Kanäle sind also zylindrisch und setzen sich in den weggeschnittenen Teil des Dreiwege-Ventils 102 spiegelsymmetrisch fort. Die Kanäle bilden im Dreiwege-Ventil 102 ein T-Stück. Das Dreiwege-Ventil 102 sitzt in einem passenden Ventilsitz 116, der dicht an dem Dreiwege-Ventil 102 anliegt und so die Kanäle abdichtet, wenn diese in dem Ventilsitz 116 gedreht sind. Im Ventilsitz 116 befinden sich zwei Durchgänge 119, durch die der größere, durchgehende Kanal im Dreiwege-Ventil 102 mit dem Rohr 103 auf der einen Seite und mit einem Einsatz 118 aus Metall zum Befestigen des Schlauchs 104 auf der anderen Seite fluiddurchlässig zu verbinden ist.

Senkrecht zu der Achse der beiden Durchgänge 119 befindet sich ein Ablasskanal 120, die den Ventilsitz 116 mit dem Inneren des nach außen geschlossenen Auffangbehälters 109 verbindet. Der Ventilsitz 116 ist einteilig aus Kunststoff mit dem Rohr 103 ausgeführt. In der geöffneten Position beziehungsweise Stellung des Dreiwege-Ventils 102 (Figur 11 unten, Figur 12 oben) ist der große durchgehende Kanal mit den beiden Durchgängen 119 verbunden und der kleine senkrechte Kanal im Dreiwege-Ventil 102 durch den Ventilsitz 116 geschlossen. Der Knochenzementteig aus der Kartusche kann dann aus dem Rohr 103 durch das Dreiwege-Ventil 102 und den Einsatz 118 in den Schlauch 104 fließen. In der geschlossenen Position beziehungsweise Stellung des Dreiwege-Ventils 102 (Figur 11 oben und Figur 12 unten) ist eine Seite des großen durchgehenden Kanals mit dem Ablasskanal 120 zum Innenraum des Auffangbehälters 109 verbunden und der kleinere senkrechte Kanal mit dem Durchgang 119 zum Schlauch 104 verbunden, während der andere Durchgang 119 zum Rohr 103 durch das Dreiwege-Ventil 102 verschlossen ist. Der Knochenzementteig kann so aus dem Schlauch 104 und gegebenenfalls aus einem an einem Luer-System-Adapter (nicht gezeigt), der am Schlauch 104 angeschlossen ist, und/oder einen angeschlossenen Trokar (nicht gezeigt) in den Auffangbehälter 109 fließen. Der Druck hierfür resultiert von einer elastischen Verformung des Schlauchs 104 und gegebenenfalls des Trokars, die sich beim Auspressen beziehungsweise beim Durchpressen des Knochenzementteigs aufgebaut hat.

Das Dreiwege-Ventil 102 kann über ein Bedienelement (nicht gezeigt), wie beispielsweise ein Knebelgriff (siehe das vorherige Ausführungsbeispiel), im Ventilsitz 116 manuell gedreht werden. Das außen zylindrische Dreiwege-Ventil 102 ist durch eine zylindrische Bohrung im Ventilsitz 116 geführt und auf der dem Bedienelement (nicht gezeigt) gegenüberliegenden Seite mit einem Stopfen (in den Figuren 11 und 12 nicht zu sehen aber analog dem vorherigen Ausführungsbeispiel ausgeführt) verbunden und so gegen Herausfallen oder versehentliches Herausziehen aus dem Ventilsitz 116 gesichert.

Durch den erfindungsgemäßen Aufbau gelingt es, dass der Strom des Knochenzementteigs durch Drehen und damit Schließen des Dreiwege-Ventils 102 schnell unterbrochen wird, ohne dass große Mengen des Knochenzementteigs durch eine Applikationsöffnung (nicht gezeigt) in den der Schlauch 104 oder der Trokar mündet, nachströmen. Gleichzeitig werden ein Austreten des Knochenzementteigs und damit eine Kontamination der Umgebung oder des Anwenders mit Hilfe des Auffangbehälters 109 verhindert, der überschüssigen Knochenzementteig aufnimmt. Des Weiteren bleibt der Druck in der Rückseite des Knochenzement-Applikators, also zwischen dem Dreiwege-Ventil 102 und dem Austragskolben der Kartusche, erhalten, so dass nach einem erneuten Öffnen des Dreiwege-Ventils 102 schnell wieder der Fluss des Knochenzementteigs bereitgestellt werden kann, ohne dass der Druck an der Rückseite Lager- und Mischvorrichtung neu aufgebaut werden muss.

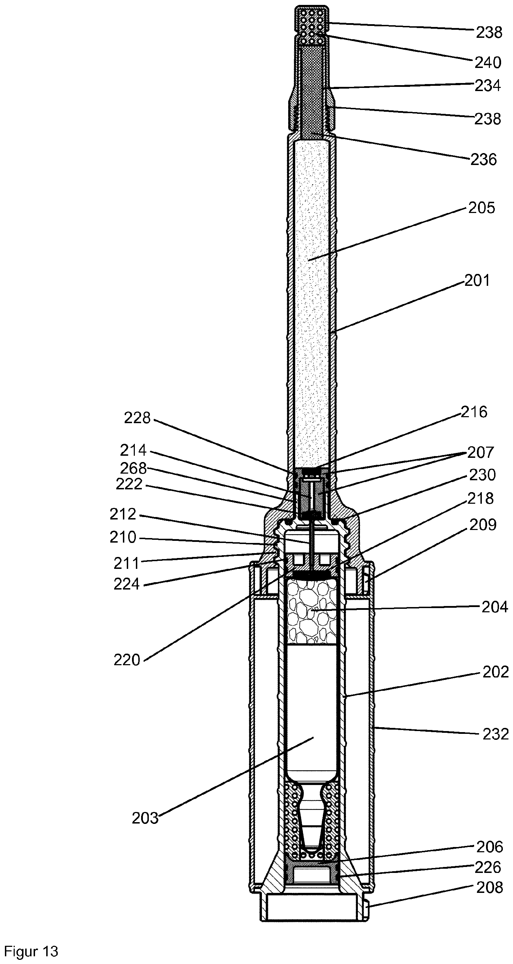

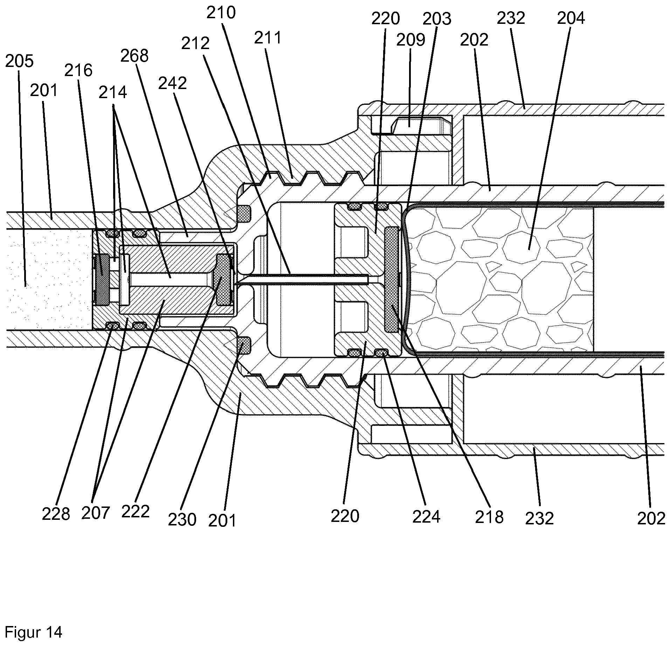

Die Figuren 13 und 14 zeigen zwei schematische Querschnittansichten einer dritten alternativen erfindungsgemäßen Lager- und Mischvorrichtung. Dabei zeigt Figur 13 eine schematische Querschnittansicht durch das dritte erfindungsgemäße Ausführungsbeispiel und Figur 14 eine Ausschnittvergrößerung der Verbindung der beiden Teile des dritten Ausführungsbeispiels nach Figur 13 vor Beginn des Auspressvorgangs als Detailansicht.

Der Aufbau der dritten alternativen erfindungsgemäßen Lager- und Mischvorrichtung gleicht dort, wo es nicht anders beschrieben oder in den Figuren 13 und 14 zu sehen ist, dem ersten Ausführungsbeispiel nach den Figuren 1 bis 10.

Die Lager- und Mischvorrichtung hat als vorderen Teil (in Figur 13 oben und in Figuren 14 links) eine Kartusche 201 aus Kunststoff mit einem zylindrischen Innenraum. Die Kartusche 201 ist mit einer Aufnahme 202 aus Kunststoff für eine Glasampulle 203 (oder Kunststoffampulle 203) lösbar verbunden. Die Aufnahme 202 weist ebenfalls einen zylindrischen Innenraum auf, in den die Glasampulle 203 eingesteckt ist. In der Glasampulle 203 befindet sich die Monomerflüssigkeit 204. In den Innenraum der Kartusche 201 ist ein Zementpulver 205 eingefüllt oder vorzugsweise eingepresst. Die Monomerflüssigkeit 204 und das Zementpulver 205 bilden die Ausgangskomponenten 204, 205 für einen PMMA-Knochenzement, der mit der Lager- und Mischvorrichtung hergestellt werden kann. Aufgrund der Glasampulle 203 kann die Monomerflüssigkeit 204 sehr lange in der Aufnahme 202 und dadurch in der Lager- und Mischvorrichtung gelagert werden.

In der Aufnahme 202 ist ein im zylindrischen Innenraum der Aufnahme 202 in Längsrichtung beweglicher Förderkolben 206 aus Kunststoff angeordnet. Der Förderkolben 206 ist im Bereich der Rückseite der Aufnahme 202 angeordnet. Die Glasampulle 203 kann mit dem Förderkolben 206 in der Aufnahme 202 zusammengedrückt und dabei zersplittert werden, indem der Förderkolben 206 in Richtung der Vorderseite, also in Richtung der Kartusche 201 gedrückt wird. Der Förderkolben 206 weist an der Vorderseite Abstreifer auf, mit denen Splitter der Glasampulle 203 von der Innenwand der Aufnahme 202 abgestreift werden.

In dem Innenraum der Kartusche 201 ist in deren Rückseite (in Figur 13 Richtung unten und in Figur 14 Richtung rechts) ein Austragskolben 207 aus Kunststoff angeordnet. An der Rückseite der Aufnahme 202 ist ein Befestigungsmittel 208 vorgesehen, mit dem die Aufnahme 202 an einer Auspressvorrichtung (in den Figuren 13 und 14 nicht dargestellt) verbunden werden kann. Ein baugleiches Befestigungsmittel 209 ist an der Rückseite der Kartusche 201 vorgesehen. Sowohl die Aufnahme 202 als auch die Kartusche 201 können also an der gleichen Auspressvorrichtung befestigt werden. Die Befestigungsmittel 208, 209 sind vorzugsweise zur Bildung eines Bajonettverschlusses geeignet und vorgesehen. Dadurch kann der Förderkolben 206, der von der Rückseite der Aufnahme 202 aus frei zugänglich ist, mit der selben Auspressvorrichtung in Richtung der Vorderseite vorgetrieben werden, wie der Austragskolben 207, der von der Rückseite der Kartusche 201 aus frei zugänglich ist, wenn die Kartusche 201 nicht mit der Aufnahme 202 verbunden ist.

Die Kartusche 201 und die Aufnahme 202 sind über ein Außengewinde 210 an der Vorderseite der Aufnahme 202 und ein Innengewinde 211 an der Rückseite der Kartusche 201 lösbar miteinander verbunden beziehungsweise lösbar miteinander verbindbar. Die Aufnahme 202 und die Kartusche 201 sind für die Monomerflüssigkeit 204 flüssigkeitsdurchlässig miteinander über eine Hohlnadel 212 aus einem Metall und eine Durchführung 214 im Austragskolben 207 verbindbar. Die Durchführung 214 durch den Austragskolben 207 mündet durch einen für das Zementpulver 205 undurchlässigen aber für die Monomerflüssigkeit 204 durchlässigen Porenfilter 216 in den Innenraum der Kartusche 201. Vor dem Eintritt in den Innenraum der Kartusche 201 verzweigt die Durchführung 214 in mehrere Arme.

In der Verbindung zur Hohlnadel 212 ist in der Aufnahme 202 ein Filter 218 angeordnet, mit dem die Splitter der Glasampulle 203 zurückgehalten werden können. Statt dem Filter 218 oder zusätzlich zum Filter 218 kann auch ein Sieb 218 vorgesehen sein. Der Filter 218 ist in einem Verbindungskolben 220 angeordnet, der beweglich im Innenraum der Aufnahme 202 angeordnet ist und an dem die Hohlnadel 212 befestigt ist. Die Hohlnadel 212 bildet dabei einen Durchgang durch den Verbindungskolben 220. Der Verbindungskolben 220 ist bei dieser Ausführung von der Vorderseite der Aufnahme 202 beabstandet. Die Verbindung zwischen dem Innenraum der Aufnahme 202 und dem Innenraum der Kartusche 201 wird bei dieser Ausführung erst durch eine Bewegung des Verbindungskolbens 220 erzeugt.

Die Hohlnadel 212 durchsticht bei einer Bewegung des Verbindungskolbens 220 durch eine elastische Membran 242 (siehe Figur 14). Wenn die Hohlnadel 212 aus der Membran 242 herausgezogen wird, dichtet die Membran 242 den Austragskolben 207 und damit die Kartusche 201 rückseitig flüssigkeitsdicht ab. Solche Membranen 242 sind von Behältern zum Aufziehen von Spritzen für den medizinischen Bereich bekannt.

Der zylindrische Verbindungskolben 220 hat einen zur Zylindergeometrie des Innenraums der Aufnahme 202 passenden Außenumfang und ist über zwei umlaufende Dichtungen 224 gegen die Innenwand der Aufnahme 202 flüssigkeitsdicht abgedichtet. Ebenso ist der Förderkolben 206 über zwei umlaufende Dichtungen 226 gegen die Innenwand der Aufnahme 202 flüssigkeitsdicht abgedichtet und der Austragskolben 207 ist über zwei umlaufende Dichtungen 228 gegen die Innenwand der Kartusche 201 flüssigkeitsdicht abgedichtet. Des Weiteren sind die Kartusche 201 und die Aufnahme 202 über eine an der Frontfläche der Aufnahme 202 befestigte Dichtung 230 gegeneinander abgedichtet, wenn die Kartusche 201 und die Aufnahme 202 aneinander befestigt sind. Alle diese Dichtungen 224, 226, 228, 230 dienen dazu, einen Austritt von Monomerflüssigkeit 204 oder von Knochenzement zu verhindern, um eine Kontamination der Umgebung (des OP-Saals und des Anwenders) zu verhindern. Die Dichtungen 224, 226, 228, 230 können hierzu aus Gummi bestehen.

Die Aufnahme 202 ist zusätzlich von einem Gehäuse 232 aus einem Kunststoff, wie Plastik, umgeben, wobei das Gehäuse 232 mit der Aufnahme 202 verbunden ist und an der Vorderseite die Befestigungsmittel 209 der Kartusche 201 abdeckt, wenn die Kartusche 201 mit der Aufnahme 202 verbunden ist. Die Kartusche 201, die Aufnahme 202 und das Gehäuse 232 können durch Spritzgießen hergestellt werden.

Die Vorderseite der Kartusche 201 mündet in ein Austragsrohr 234, das ein Außengewinde aufweist. Im Inneren der Austragsrohrs 234 ist ein Porenfilter 236 angeordnet, der für das Zementpulver 205 undurchlässig aber für Gase durchlässig ist. An dem Außengewinde des Austragsrohrs 234 ist eine Kappe 238 befestigt, wobei der vordere Teil der Kappe 238 mit einem Styropor oder Schaumstoff 240 gefüllt ist. Die Kappe 238 kann von dem Austragsrohr 234 abgeschraubt werden kann. Die Kappe 238 weist seitliche Öffnungen auf. Durch diesen Aufbau kann das Innere der Kartusche 201 und das Zementpulver 205 mit Hilfe von Ethylenoxid sterilisiert werden, da die Öffnungen in der Kappe 238, das Styropor oder der Schaumstoff 240, der Porenfilter 236 und die Zwischenräume zwischen den Pulverpartikeln des Zementpulvers 205 luftdurchlässig sind. Gleichzeitig kann Luft aus der Aufnahme durch das Zementpulver 205, den Porenfilter 236, das Styropor oder der Schaumstoff 240 und die Öffnungen in der Kappe 238 herausgepresst werden, wenn der Förderkolben 206 in Richtung der Aufnahme 201 gepresst wird.

Das Zementpulver 205 ist in der Kartusche 201 eingeschlossen, da alle Öffnungen mit Hilfe von den Porenfiltern 216, 236 für das Zementpulver 205 undurchlässig abgeschlossen sind. Der Inhalt der Kartusche 201 kann dabei durch Evakuieren und Spülen mit Ethylenoxid sterilisiert werden. Dadurch ist die Lager- und Mischvorrichtung auch zum langfristigen Lagern des Zementpulvers 205 geeignet.

Im Folgenden wird der Ablauf eines erfindungsgemäßen Verfahrens anhand des dritten Ausführungsbeispiels diskutiert. Zu Beginn des Verfahrens liegt die Lager- und Mischvorrichtung im Ausgangszustand vor, so wie sie auch in den Figuren 13 und 14 gezeigt ist. Die Kartusche 201 und die Aufnahme 202 der Lager- und Mischvorrichtung sind also zunächst miteinander über die Gewinde 210, 211 miteinander verbunden. In diesem Zustand wird die Lager- und Mischvorrichtung in eine Auspressvorrichtung in Form einer herkömmlichen Kartuschenpistole eingesetzt und mit dem Befestigungsmittel 208 an der Auspressvorrichtung befestigt.

Die Auspressvorrichtung umfasst einen linear vortreibbaren Stößel und ist analog der Auspressvorrichtung 43 nach den Figuren 1 bis 10 ausgeführt. Die Auspressvorrichtung ist über ein Gegenbefestigungsmittel an die Rückseite der Aufnahme 202 angeschlossen, so dass beim Vortreiben des Stößels dieser auf den Förderkolben 206 drückt und diesen in Richtung der Kartusche 201 vortreibt. Hierzu ist der Stößel gegen das Gegenbefestigungsmittel und damit gegen die Aufnahme 202 linear beweglich gelagert.

Die Auspressvorrichtung wird bedient und dabei der Stößel und mit dem Stößel der Förderkolben 206 in Richtung der Kartusche 201 vorgetrieben. Da die Glasampulle 203 oder Kunststoffampulle 203 an der Vorderseite an dem Verbindungskolben 220 anliegt, wird der Verbindungskolben 220 in Richtung der Kartusche 201 gedrückt. Die Hohlnadel 212 durchsticht die Membran 242 und einen Pfropfen 222. Der Pfropfen 222 dient der Stabilisierung der Hohlnadel 212. Dadurch wird eine flüssigkeitsleitende Verbindung zwischen dem Innenraum der Kartusche 201 und dem Innenraum der Aufnahme 202 hergestellt. Durch die Bewegung des Förderkolbens 206 werden Belüftungsöffnungen (nicht gezeigt) vom Förderkolben 206 verschlossen, die in der Wandung der Aufnahme 202 im Bereich des Förderkolbens 206 angeordnet sind und durch die der Innenraum der Aufnahme 202 im Ausgangszustand evakuierbar und mit Ethylenoxid sterilisierbar ist. Der Verbindungkolben 220 wird an die Vorderseite des Innenraums der Aufnahme 202 gedrückt und kann nicht weiterbewegt werden. Dadurch verkleinert sich der Innenraum der Aufnahme 202 beim weiteren Vortreiben des Förderkolbens 206 und die Glasampulle 203 zerbricht und die Monomerflüssigkeit 204 tritt aus der Glasampulle 203 in den Innenraum der Aufnahme 202 aus. Überstehende Luft aus der Aufnahme 202 wird durch den Filter 218, die Durchführung 214, den Porenfilter 216, durch die Zwischenräume des Zementpulvers 205, durch den Porenfilter 236, durch den Schaumstoff 240 und aus den Öffnungen in der Kappe 238 aus der Lager- und Mischvorrichtung heraus gedrückt.

Von der Glasampulle 203 bleiben schließlich nur kleine Splitter zurück, die von dem Filter 218 zurückgehalten werden und in der Aufnahme 202 verbleiben. Die Monomerflüssigkeit 204 wird durch den Filter 218, die Durchführung 214 und den Porenfilter 216 in das Zementpulver 205 gepresst und beginnt dort mit dem Zementpulver 205 zu reagieren, so dass sich aus dem Gemisch der Knochenzementteig bildet. In diesem Zustand wird die Lager- und Mischvorrichtung aus der Auspressvorrichtung entnommen. Die Aufnahme 202 und das Gehäuse 232 werden von der Kartusche 201 abgeschraubt. Zudem wird die Kappe 238 mit dem Porenfilter 238 und dem Schaumstoff 240 abgeschraubt und eine verlängerte Austragsöffnung auf das Austragsrohr 234 aufgeschraubt. Anschließend wird die Kartusche 201 mit der verlängerten Austragsöffnung wieder an der Auspressvorrichtung befestigt. Zuvor wurde die Auspressvorrichtung zurückgesetzt, das heißt, der Stößel wurde wieder zurückgeschoben und in die Ausgangsstellung überführt. Nach dem Herausziehen der Hohlnadel 212 verschließt sich die Membran 242 von selbst, so dass keine Monomerflüssigkeit 204, die sich noch in der Durchführung 214 befindet, an der Rückseite der Kartusche 201 austreten kann, wenn die Kartusche 201 von der Aufnahme 202 getrennt ist.

Zur Befestigung der Auspressvorrichtung an der Kartusche 201 greifen die Gegenbefestigungsmittel der Auspressvorrichtung in die Befestigungsmittel 209 an der Rückseite der Kartusche 201, die auch schon zur Befestigung an den Befestigungsmitteln 208 der Aufnahme 202 verwendet wurden. Da die Befestigungsmittel 209 an der Kartusche 201 und die Befestigungsmittel 208 an der Aufnahme 202 gleich sind, können beide am selben Gegenbefestigungsmittel der selben Auspressvorrichtung befestigt werden. Dies vereinfacht den Aufbau und ermöglicht die Verwendung herkömmlicher Auspressvorrichtungen. Die Befestigungsmittel 208, 209 und das Gegenbefestigungsmittel bilden dabei vorzugsweise einen Bajonettverschluss.

Die verlängerte Austragsöffnung kann vorzugsweise ein Dreiwege-Ventil analog der ersten oder der zweiten Ausführung aufweisen. Ebenso kann an dem Austragsrohr 234 ein Schlauch und/oder ein Trokar angeschlossen werden.

Der abgeschraubte rückseitige Teil der Lager- und Mischvorrichtung, nämlich die Aufnahme 202 mit den Splittern der Glasampulle 203 darin und das Gehäuse 232, kann nach dem Abschrauben entsorgt werden. An der Vorderseite der Aufnahme ist ein Schutzrohr 268 vorgesehen, in dem die Hohlnadel 212 angeordnet ist, so dass sich der Anwender nicht so leicht an der Hohlnadel 212 verletzen kann.

Über den Schlauch und den Trokar oder über das Austragsrohr 234 kann der Knochenzementteig ausgetragen werden. Dazu wird der Austragskolben 207 mit dem Stößel in Richtung des Austragsrohrs 234 vorgetrieben. Der Knochenzementteig aus dem Inneren der Kartusche 201 wird entweder durch das Austragsrohr 234 direkt oder bei geöffnetem Dreiwege-Ventil durch das Austragsrohr 234, durch das Dreiwege-Ventil, durch den Schlauch und den Trokar ausgetrieben und kann dort an den Wirbeln eines Patienten appliziert werden oder theoretisch zur weiteren Verarbeitung verwendet werden. Zur Unterbrechung des Flusses des Knochenzementteigs kann gegebenenfalls das Dreiwege-Ventil bedient werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 201: Kartusche
- 2, 202: Aufnahme
- 3, 203: Ampulle
- 4, 204: Monomerflüssigkeit
- 5, 205: Zementpulver
- 6, 206: Förderkolben
- 7,207: Austragskolben
- 8, 208: Befestigungsmittel / Bajonettverschluss
- 9, 209: Befestigungsmittel / Bajonettverschluss
- 10,210: Außengewinde
- 11, 211: Innengewinde
- 12: Röhrchen
- 14,214: Durchführung
- 16,216: Porenfilter
- 18,218: Filter
- 20,220: Verbindungskolben
- 22: Dichtlippe
- 24, 224: Dichtung
- 26, 226: Dichtung
- 28, 228: Dichtung
- 30,230: Dichtung
- 32, 232: Gehäuse
- 34, 234: Austragsrohr
- 36, 236: Porenfilter
- 38, 238: Kappe
- 40, 240: Schaumstoff
- 42: Flügel
- 43: Auspressvorrichtung / Kartuschenpistole
- 44: Stößel
- 46: Teller
- 48: Gegenbefestigungsmittel / Bajonettverschluss
- 50: Lagerung
- 52: Splitter
- 54: Knochenzementteig / Gemisch
- 56: Dreiwege-Ventil
- 58: Knebelgriff
- 59: Ventilsitz / Rohr
- 60: Auffangbehälter
- 61: Durchgang
- 62, 78: Flügel
- 64: Schlauch
- 66: Trokar
- 68, 268: Schutzrohr
- 70, 74: Luer-System-Adapter
- 72: Anschluss
- 76: Schlauch
- 80: Einsatz
- 82: Hülse
- 84: Dichtung
- 86: Stopfen
- 88: Ablasskanal
- 90: Belüftungsöffnung
- 102: Dreiwege-Ventil
- 103: Rohr
- 104: Schlauch
- 109: Auffangbehälter
- 112: Hülse
- 114: Statischer Mischer
- 116: Ventilsitz
- 118: Einsatz
- 119: Durchgang
- 120: Ablasskanal
- 212: Hohlnadel
- 222: Pfropfen
- 242: Membran

## Patentansprüche

1. Lager- und Mischvorrichtung zur Herstellung eines Knochenzementteigs (54) aus einer Monomerflüssigkeit (4, 204) und einem Zementpulver (5, 205) als Ausgangskomponenten des Knochenzements, die Lager- und Mischvorrichtung aufweisend
eine Aufnahme (2, 202), in der ein Monomerflüssigkeitsbehälter (3, 203) angeordnet ist, wobei in dem Monomerflüssigkeitsbehälter (3, 203) die Monomerflüssigkeit (4, 204) enthalten ist, und
eine Kartusche (1, 201), in der das Zementpulver (5, 205) enthalten ist, wobei in der Aufnahme (2, 202) ein in Längsrichtung der Aufnahme (2, 202) beweglicher Förderkolben (6, 206) angeordnet ist, der von einer Rückseite der Aufnahme (2, 202) aus zugänglich ist, wobei
in der Kartusche (1, 201) ein in Längsrichtung im Inneren der Kartusche (1, 201) verschiebbarer Austragskolben (7, 207) angeordnet ist, wobei die Vorderseite der Aufnahme (2, 202) mit der Rückseite der Kartusche (1, 201) derart verbunden oder verbindbar ist, dass ein Innenraum der Aufnahme (2, 202) mit einem Innenraum der Kartusche (1, 201) für die Monomerflüssigkeit (4, 204) durchlässig verbunden ist, wobei die Verbindung lösbar ist, und wobei
an der Rückseite der Aufnahme (2, 202) ein erstes Befestigungsmittel (8, 208) vorgesehen ist und an der Rückseite der Kartusche (1, 201) ein zweites Befestigungsmittel (9, 209) vorgesehen ist, wobei das erste Befestigungsmittel (8, 208) und das zweite Befestigungsmittel (9, 209) gleich oder gleichartig sind, **dadurch gekennzeichnet, dass**
an der Rückseite der Kartusche (1, 201), insbesondere im Austragskolben (7, 207) der Kartusche (1, 201), eine verschließbare Durchführung (14, 214) in den Innenraum der Kartusche (1, 201) vorgesehen ist.

2. Lager- und Mischvorrichtung nach Anspruch 1, **dadurch gekennzeichnet, dass** die verschließbare Durchführung (14, 214) beim Lösen der flüssigkeitsdurchlässigen Verbindung zwischen der Aufnahme (2, 202) und der Kartusche (1, 201) flüssigkeitsdicht verschließbar ist, wobei besonders bevorzugt die verschließbare Durchführung (14, 214) ein Ventil, zumindest zwei Dichtlippen (22) oder eine selbstschließende durchstechbare Membran (242) aufweist.

3. Lager- und Mischvorrichtung nach Anspruch 1 oder 2, **dadurch gekennzeichnet, dass** an der Vorderseite der Aufnahme (2, 202) eine Hohlnadel (212) oder ein Röhrchen (12) vorgesehen ist, mit dem das Innere der Aufnahme (2, 202) flüssigkeitsleitend mit dem Innenraum der Kartusche (1, 201) verbindbar ist, wobei die Hohlnadel (212) oder das Röhrchen (12) mit der verschließbaren Durchführung (14, 214) die für die Monomerflüssigkeit (4, 204) durchlässige Verbindung in den Innenraum der Kartusche (1, 201) bildet, wenn die Aufnahme (2, 202) mit der Kartusche (1, 201) verbunden ist.

4. Lager- und Mischvorrichtung nach einem der vorangehenden Ansprüche, **dadurch gekennzeichnet, dass**
an der Vorderseite der Aufnahme (2, 202) ein Verbindungskolben (20, 220) angeordnet ist, der in Längsrichtung beweglich im Inneren der Aufnahme (2, 202) angeordnet ist, wobei der Monomerflüssigkeitsbehälter (3, 203) zwischen dem Verbindungskolben (20, 220) und dem Förderkolben (6, 206) angeordnet ist, wobei in dem Verbindungskolben (20, 220) ein Durchgang vorgesehen ist, der für die Monomerflüssigkeit (4, 204) und für Gase durchlässig ist und mit dem eine Flüssigkeitsverbindung zu dem Innenraum der Kartusche (1, 201) herstellbar ist, wobei vorzugsweise durch eine Bewegung des Verbindungskolbens (20, 220) in Richtung der Kartusche (1, 201) eine mit der Vorderseite der Aufnahme (2, 202) verbundenen Kartusche (1, 201), die verschließbare Durchführung (14, 214) an der Rückseite der Kartusche (1, 201) oder ein Verschlussmittel (12, 212), das die flüssigkeitsdurchlässige Verbindung zwischen dem Innenraum der Kartusche (1, 201) und dem Innenraum der Aufnahme (2, 202) verschließt, zu öffnen ist.

5. Lager- und Mischvorrichtung nach einem der vorangehenden Ansprüche, **dadurch gekennzeichnet, dass**
die Lager- und Mischvorrichtung ein Knochenzement-Applikator zum Lagern und Mischen der Ausgangskomponenten und zur Applikation des Knochenzementteigs (54) im Bereich der Wirbelsäule ist, wobei der Knochenzement-Applikator eine verlängerte Austragsöffnung aufweist, die an der Vorderseite der Kartusche (1, 201) angeordnet ist, wobei besonders bevorzugt die verlängerte Austragsöffnung durch ein Austragsrohr (34, 234), einen Schlauch (64, 104) und/oder einen Trokar (66) realisiert ist.

6. Lager- und Mischvorrichtung nach Anspruch 5, **dadurch gekennzeichnet, dass** ein von außen bedienbares Dreiwege-Ventil (56, 102) in der verlängerten Austragsöffnung oder zwischen der verlängerten Austragsöffnung und der Kartusche (1, 201) angeordnet ist,
ein Auffangbehälter (60, 109) zum Aufnehmen von Knochenzementteig (54) an dem Dreiwege-Ventil (56, 102) angeordnet ist, wobei die verlängerte Austragsöffnung in eine Applikationsöffnung mündet, die an dem von der Kartusche (1, 201) abgewandten Ende der verlängerten Austragsöffnung angeordnet ist, wobei das Dreiwege-Ventil (56, 102) derart aufgebaut ist, dass es
in einer ersten Stellung eine Fluidverbindung zwischen der Applikationsöffnung und der Kartusche (1, 201) bereitstellt und einen Ablasskanal (88, 120) zum Auffangbehälter (60, 109) verschließt und
in einer zweiten Stellung eine Fluidverbindung zwischen der Applikationsöffnung und dem Auffangbehälter (60, 109) bereitstellt und einen Durchgang (61, 119) zur Kartusche (1, 201) verschließt.

7. Lager- und Mischvorrichtung nach einem der vorangehenden Ansprüche, **dadurch gekennzeichnet, dass**
die Aufnahme (2, 202) ein Verbindungsmittel (12, 212) aufweist, insbesondere zumindest eine Hohlnadel (212) oder zumindest ein Röhrchen (12) als Verbindungsmittel (12, 212) aufweist, wobei das Verbindungsmittel (12, 212) an der Vorderseite der Aufnahme (2, 202) angeordnet ist und die Aufnahme (2, 202) über das Verbindungsmittel (12, 212) flüssigkeitsleitend mit dem Innenraum der Kartusche (1, 201) verbunden oder verbindbar ist und wobei die Aufnahme (2, 202) bis auf das Verbindungsmittel (12, 212) flüssigkeitsdicht geschlossen ist.

8. Lager- und Mischvorrichtung nach einem der vorangehenden Ansprüche, **dadurch gekennzeichnet, dass**
der Monomerflüssigkeitsbehälter (3, 203) eine Glasampulle (3, 203), eine Kunststoffampulle (3, 203) ein Kunststoff-Folienbeutel oder ein Aluminium-Kunststoff-Verbund-Beutel ist, die oder der im Inneren der Aufnahme (2, 202) zu öffnen ist, und/oder
der Querschnitt des Innenraums der Kartusche (1, 201) maximal 4 cm² beträgt, bevorzugt maximal 2,5 cm² beträgt, besonders bevorzugt maximal 1,2 cm² beträgt.

9. Lager- und Mischvorrichtung nach einem der vorangehenden Ansprüche, **dadurch gekennzeichnet, dass**
das erste Befestigungsmittel (8, 208) und das zweite Befestigungsmittel (9, 209) an einer einzigen Halterung (48) einer Auspressvorrichtung (43), insbesondere einer Kartuschenpistole, zu befestigen sind.

10. Lager- und Mischvorrichtung nach einem der vorangehenden Ansprüche, **dadurch gekennzeichnet, dass**
die Kartusche (1, 201) an der Vorderseite mit einem Kartuschenkopf abgeschlossen ist, wobei sich im Kartuschenkopf eine Auslassöffnung befindet und die Auslassöffnung mit einem für das Zementpulver (5, 205) in der Kartusche (1, 201) undurchlässigen und für Gas durchlässigen Verschluss (36, 236), insbesondere einem Porenfilter (36, 236), verschlossen ist, wobei vorzugsweise der Verschluss (36, 236) durch axiale Druckbeanspruchung oder durch manuelle Krafteinwirkung zu öffnen ist, wobei die Aufnahme (2, 202) auf der Rückseite durch den Förderkolben (6, 206) flüssigkeitsdicht geschlossen oder verschließbar ist, vorzugsweise flüssigkeitsdicht und gasdicht geschlossen oder verschließbar ist.

11. Lager- und Mischvorrichtung nach einem der vorangehenden Ansprüche, **dadurch gekennzeichnet, dass**
die Kartusche (1, 201) hohlzylinderförmig ist und das zweite Befestigungsmittel (9, 209) zur Verbindung mit einer Auspressvorrichtung (43) vorgesehen ist, wobei ein Kartuschenkopf die Vorderseite der hohlzylinderförmigen Kartusche (1, 201) abschließt, wobei eine Auslassöffnung im Kartuschenkopf angeordnet ist, und wobei die Auslassöffnung die Außenseite des Kartuschenkopfs mit der Innenseite des Kartuschenkopfs gasdurchlässig verbindet, wobei ein Austragsrohr (34, 234) am Kartuschenkopf an der Auslassöffnung angeschlossen ist, wobei eine durchstechbare Membran (242) vorgesehen ist, welche die Rückseite des Austragskolbens (7, 207) flüssigkeitsundurchlässig verschließt, und
die Aufnahme (2, 202) hohlzylinderförmig ist, wobei in einem Verbindungskolben (20, 220) in der Aufnahme (2, 202) eine für Gase und Flüssigkeiten durchlässige poröse Scheibe (18, 218) vorgesehen ist und mindestens eine an der Vorderseite eines Verbindungskolbens (20, 220) angeordnete Hohlnadel (212), die über einen Kanal mit der Rückseite der porösen Scheibe (18, 218) flüssigkeitsdurchlässig verbunden ist.

12. Lager- und Mischvorrichtung nach einem der vorangehenden Ansprüche, **dadurch gekennzeichnet, dass**
das Volumen der Monomerflüssigkeit (4, 204) im Monomerflüssigkeitsbehälter (3, 203) mindestens so groß ist wie das Volumen der mit Luft gefüllten Zwischenräume zwischen den Zementpulverpartikeln in der Kartusche (1, 201), bevorzugt mindestens so groß ist wie das Volumen der Flüssigkeitsleitungen (12, 14, 16, 18, 212, 214, 216, 218) zwischen dem Innenraum der Kartusche (1, 201) und dem Innenraum der Aufnahme (2, 202) plus das Volumen der mit Luft gefüllten Zwischenräume zwischen den Zementpulverpartikeln in der Kartusche (1, 201), und in der Wandung der Aufnahme (2, 202) zumindest eine Belüftungsöffnung (90) angeordnet ist, die den Innenraum der Aufnahme (2, 202), in dem der Monomerflüssigkeitsbehälter (3, 203) angeordnet ist, mit der Umgebung verbindet, wobei bevorzugt die zumindest eine Belüftungsöffnung (90) derart dicht im Bereich des Förderkolbens (6, 206) angeordnet ist, dass sie durch eine Bewegung des Förderkolbens (6, 206) in Richtung der Vorderseite der Aufnahme (2, 202) verschlossen wird, bevor der Monomerflüssigkeitsbehälter (3, 203) durch die Bewegung des Förderkolbens (6, 206) geöffnet ist.

13. Verfahren zur Herstellung eines Knochenzementteigs (54), insbesondere eines pastenförmigen Polymethylmethacrylat-Knochenzementteigs (54), wobei der Knochenzementteig (54) aus einem Zementpulver (5, 205) und einer Monomerflüssigkeit (4, 204) mit einer Lager- und Mischvorrichtung hergestellt wird, wobei die Lager- und Mischvorrichtung eine Aufnahme (2, 202) enthaltend einen Monomerflüssigkeitsbehälter (3, 203) mit der Monomerflüssigkeit (4, 204) darin und eine Kartusche (1, 201) enthaltend das Zementpulver (5, 205) umfasst, wobei die Aufnahme (2, 202) an der Rückseite der Kartusche (1, 201) befestigt ist oder befestigt wird, wobei an der Rückseite der Kartusche (1, 201) eine verschließbare Durchführung (14, 214) vorgesehen ist, **gekennzeichnet durch** die folgenden nacheinander ablaufenden Schritte:
a) Einsetzen der Lager- und Mischvorrichtung in eine Auspressvorrichtung (43), die Auspressvorrichtung (43) aufweisend einen axial vortreibbaren Stößel (44),
b) ein Förderkolben (6, 206), der innerhalb der Aufnahme (2, 202) an deren Rückseite beweglich gelagert ist, wird mit dem Stößel (44) in Richtung der Kartusche (1, 201) vorgetrieben, wobei durch die Bewegung des Förderkolbens (6, 206) der Monomerflüssigkeitsbehälter (3, 203) geöffnet wird und die Monomerflüssigkeit (4, 204) aus dem Monomerflüssigkeitsbehälter (3, 203) durch die verschließbare Durchführung (14, 214) in die Kartusche (1, 201) gepresst wird, wobei im Innenraum der Kartusche (1, 201) sich das Zementpulver (5, 205) mit der Monomerflüssigkeit (4, 204) mischt,
c) die Aufnahme (2, 202) wird von der Kartusche (1, 201) getrennt, wobei die verschließbare Durchführung (14, 214) verschlossen wird oder sich beim Trennen selbsttätig verschließt und wobei die Kartusche (1, 201) in der Auspressvorrichtung (43) verbleibt oder nach der Trennung in die Auspressvorrichtung (43) eingesetzt wird,
d) ein Austragskolben (7, 207), der innerhalb der Kartusche (1, 201) an deren Rückseite beweglich gelagert ist, wird mit dem Stößel (44) in Richtung der Vorderseite der Kartusche (1, 201) vorgetrieben, wobei durch die Bewegung des Förderkolbens (6, 206) die Mischung aus dem Zementpulver (5, 205) und der Monomerflüssigkeit (4, 204) aus der Kartusche (1, 201) als Knochenzementteig (54) ausgetrieben wird.

14. Verfahren nach Anspruch 13, **dadurch gekennzeichnet, dass**
das Verfahren mit einer Lager- und Mischvorrichtung nach einem der Ansprüche 1 bis 12 umgesetzt wird.

15. Verfahren nach einem der Ansprüche 13 oder 14, **dadurch gekennzeichnet, dass** an der Rückseite der Aufnahme (2, 202) ein erstes Befestigungsmittel (8, 208) vorgesehen ist und an der Rückseite der Kartusche (1, 201) ein zweites Befestigungsmittel (9, 209) vorgesehen ist, wobei beim Einsetzen der Lager- und Mischvorrichtung in Schritt a) die Lager- und Mischvorrichtung über das zweite Befestigungsmittel (9, 209) an einer Halterung (48) der Auspressvorrichtung (43) befestigt wird und in Schritt c) die Kartusche (1, 201) mit dem ersten Befestigungsmittel (8, 208) an der Halterung (48) der Auspressvorrichtung (43) befestigt wird.

16. Verfahren nach einem der Ansprüche 13 bis 15, **dadurch gekennzeichnet, dass** an der Vorderseite der Aufnahme (2, 202) ein Verbindungskolben (20, 220) angeordnet ist, wobei der Monomerflüssigkeitsbehälter (3, 203) zwischen dem Verbindungskolben (20, 220) und dem Förderkolben (6, 206) angeordnet ist, wobei in Schritt b) beim Vortreiben des Förderkolbens (6, 206) vor dem Einpressen der Monomerflüssigkeit (4, 204) in die Kartusche (1, 201) der Verbindungskolben (20, 220) in Richtung der Kartusche (1, 201) getrieben wird, wobei dabei ein Durchgang in die Kartusche (1, 201) geöffnet wird, so dass der Innenraum der Kartusche (1, 201) und der Innenraum der Aufnahme (2, 202) zwischen dem Förderkolben (6, 206) und dem Verbindungskolben (20, 220) flüssigkeitsdurchlässig miteinander verbunden werden.

17. Verfahren nach Anspruch 16, **dadurch gekennzeichnet, dass**
bei oder nach dem flüssigkeitsdurchlässigen Verbinden des Innenraums der Kartusche (1, 201) und des Innenraums der Aufnahme (2, 202) der Monomerflüssigkeitsbehälter (3, 203) durch eine Bewegung des Förderkolbens (6, 206) gegen den Verbindungskolben (20, 220) geöffnet wird, insbesondere eine Kunststoff- oder Glasampulle (3, 203) als Monomerflüssigkeitsbehälter (3, 203) zwischen dem Förderkolben (6, 206) und dem Verbindungskolben (20, 220) zerdrückt wird, so dass die Monomerflüssigkeit (4, 204) im Innenraum der Aufnahme (2, 202) zum Einpressen in die Kartusche (1, 201) verfügbar ist.

18. Verfahren nach einem der Ansprüche 13 bis 17, **dadurch gekennzeichnet, dass** ein von außen bedienbares Dreiwege-Ventil (56, 102) in der verlängerten Austragsöffnung oder hinter der Kartusche (1, 201) angeordnet ist, wobei das Dreiwege-Ventil (56, 102) in eine erste Stellung gebracht wird oder sich in der ersten Stellung befindet, wobei das Dreiwege-Ventil (56, 102) in der ersten Stellung eine durchgängige Verbindung von dem Innenraum der Kartusche (1, 201) durch die verlängerte Austragsöffnung bereitstellt, und es erfolgt in Schritt d) ein Auspressen der Kartusche (1, 201) mit Hilfe der Auspressvorrichtung (43), wobei der aus dem Zementpulver (5, 205) und der Monomerflüssigkeit (4, 204) gemischte Knochenzementteig (54) durch das Dreiwege-Ventil (56, 102) und durch die verlängerte Austragsöffnung gedrückt wird, und ein nachfolgender Schritt e) erfolgt, in dem das Dreiwege-Ventil (56, 102) in ein zweite Stellung überführt wird, wobei das Dreiwege-Ventil (56, 102) in der zweiten Stellung den Fluss aus der Kartusche (1, 201) durch das Dreiwege-Ventil (56, 102) stoppt und ein Teil des hinter dem Dreiwege-Ventil (56, 102) in der verlängerten Austragsöffnung unter Druck stehenden Knochenzementteigs (54) durch das Dreiwege-Ventil (56, 102) in einen Auffangbehälter (60, 109) gedrückt wird, wobei
nach Schritt e) in einem Schritt f) das Dreiwege-Ventil (56, 102) wieder in die erste Stellung überführt wird und dadurch der Knochenzementteig (54) wieder durch das Dreiwege-Ventil (56, 102) durch die verlängerte Austragsöffnung geleitet wird, wobei bevorzugt anschließend die Schritte d), e) und f) chronologisch einmal oder mehrmals wiederholt werden.

## Claims

1. Storage and mixing device for the production of a bone cement dough (54) from a monomer liquid (4, 204) and a cement powder (5, 205) as starting components of the bone cement, the storage and mixing device comprising
a receptacle (2, 202), in which a monomer liquid container (3, 203) is arranged, whereby the monomer liquid container (3, 203) contains the monomer liquid (4, 204), a cartridge (1, 201) containing the cement powder (5, 205),
a feed plunger (6, 206) which is arranged in the receptacle (2, 202), whereby the feed plunger (6, 206) is movable in a longitudinal direction of the receptacle (2, 202) and is accessible from a rear side of the receptacle (2, 202), and
a dispensing plunger (7, 207) which is arranged in the cartridge (1, 201), whereby the dispensing plunger (7, 207) is shiftable in a longitudinal direction in the inside of the cartridge (1, 201), whereby
the front side of the receptacle (2, 202) is connected or connectable to the rear side of the cartridge (1, 201) such that an internal space of the receptacle (2, 202) is connected to an internal space of the cartridge (1, 201) such as to be permeable to the monomer liquid (4, 204), whereby the connection is detachable, and whereby a first securing means (8, 208) is provided on the rear side of the receptacle (2, 202) and a second securing means (9, 209) is provided on the rear side of the cartridge (1, 201), whereby the first securing means (8, 208) and the second securing means (9, 209) are identical or congeneric, **characterized in that**
a closable feedthrough (14, 214) into the internal space of the cartridge (1, 201) is provided on the rear side of the cartridge (1, 201), in particular in the dispensing plunger (7, 207) of the cartridge (1, 201).

2. Storage and mixing device according to claim 1, **characterised in that**
the closable feedthrough (14, 214) is closable in liquid tight manner upon the liquid-permeable connection between the receptacle (2, 202) and the cartridge (1, 201) being detached, whereby the closable feedthrough (14, 214) particularly preferably comprises a valve, at least two sealing lips (22) or a self-closing puncturable membrane (242).

3. Storage and mixing device according to claim 1 or 2, **characterised in that**
a hollow needle (212) or a tube (12) is provided on the front side of the receptacle (2, 202) by which the inside of the receptacle (2, 202) is connectable to the internal space of the cartridge (1, 201) in liquid-conducting manner, whereby the hollow needle (212) or the tube (12) together with the closable feedthrough (14, 214) form the monomer liquid (4, 204) permeable connection into the internal space of the cartridge (1, 201), if the receptacle (2, 202) is connected to the cartridge (1, 201).

4. Storage and mixing device according to any one of the preceding claims, **characterised in that**
a connecting plunger (20, 220) is arranged on the front side of the receptacle (2, 202), and is arranged such as to be mobile in longitudinal direction in the inside of the receptacle (2, 202), whereby the monomer liquid container (3, 203) is arranged between the connecting plunger (20, 220) and the feed plunger (6, 206), whereby a passage is provided in the connecting plunger (20, 220) and the passage is permeable to the monomer liquid (4, 204) and gases and is usable to establish a fluid connection to the internal space of the cartridge (1, 201), whereby, preferably, a motion of the connecting plunger (20, 220) in the direction of the cartridge (1, 201) a cartridge (1, 201) that is connected to the front side of the receptacle (2, 202), the closable feedthrough (14, 214) on the rear side of the cartridge (1, 201) or a closing means (12, 212) that closes the liquid-permeable connection between the internal space of the cartridge (1, 201) and the internal space of the receptacle (2, 202) is openable.

5. Storage and mixing device according to any one of the preceding claims, **characterised in that**
the storage and mixing device is a bone cement applicator for storage and mixing of the starting components and for application of the bone cement dough (54) in the area of the spine, whereby the bone cement applicator comprises an extended dispensing opening that is arranged at the front side of the cartridge (1, 201), whereby the extended dispensing opening particularly preferably is implemented by means of a dispensing tube (34, 234), a hose (64, 104) and/or a trocar (66).

6. Storage and mixing device according to claim 5, **characterised in that**
a three-way valve (56, 102) that is operable from outside is arranged in the extended dispensing opening or between the extended dispensing opening and the cartridge (1, 201),
a collecting container (60, 109) for reception of bone cement dough (54) is arranged on the three-way valve (56, 102), whereby the extended dispensing opening merges into an application opening that is arranged on the end of the extended dispensing opening that faces away from the cartridge (1, 201), whereby the three-way valve (56, 102) is designed appropriately such that it,
being in a first position, provides a fluid connection between the application opening and the cartridge (1, 201) and closes a discharge channel (88, 120) toward the collecting container (60, 109) and,
being in a second position, provides a fluid connection between the application opening and the collecting container (60, 109) and closes a passage (61, 119) toward the cartridge (1, 201).

7. Storage and mixing device according to any one of the preceding claims, **characterised in that**
the receptacle (2, 202) comprises a connecting means (12, 212), in particular comprises at least one hollow needle (212) or at least one tube (12) as connecting means (12, 212), whereby the connecting means (12, 212) is arranged on the front side of the receptacle (2, 202) and the receptacle (2, 202) is connected or connectable to the internal space of the cartridge (1, 201) through the connecting means (12, 212) in liquid-conducting manner, and whereby the receptacle (2, 202) is closed in liquid-tight manner except for the connecting means (12, 212).

8. Storage and mixing device according to any one of the preceding claims, **characterised in that**
the monomer liquid container (3, 203) is a glass ampoule (3, 203), a plastic ampoule (3, 203), a plastic film bag or an aluminium-plastic compound bag which is openable in the inside of the receptacle (2, 202) and/or
the cross-section of the internal space of the cartridge (1, 201) is at most 4 cm², preferably at most 2.5 cm², particularly preferably at most 1.2 cm².

9. Storage and mixing device according to any one of the preceding claims, **characterised in that**
the first securing means (8, 208) and the second securing means (9, 209) is securable to a single holder (48) of an extrusion device (43), in particular of a cartridge gun.

10. Storage and mixing device according to any one of the preceding claims, **characterised in that**
the cartridge (1, 201) is closed on the front side by a cartridge head, whereby an outlet opening is situated in the cartridge head and the outlet opening is closed by a closure (36, 236) which is impermeable to the cement powder (5, 205) in the cartridge (1, 201) and which is permeable to gas, in particular a pore filter (36, 236), whereby the closure (36, 236) preferably is openable through an axial pressure load or by action of a manual force, whereby
the receptacle (2, 202) is closed or closable on the rear side by the feed plunger (6, 206) in liquid-tight manner, preferably is closed or closable in liquid-tight and gas-tight manner.

11. Storage and mixing device according to any one of the preceding claims, **characterised in that**
the cartridge (1, 201) is hollow cylinder-shaped and the second securing means (9, 209) is provided to be connected to an extrusion device (43), whereby a cartridge head closes the front side of the hollow cylinder-shaped cartridge (1, 201), whereby an outlet opening is arranged in the cartridge head, and whereby the outlet opening connects the outside of the cartridge head to the inside of the cartridge head in gas-permeable manner, whereby a dispensing tube (34, 234) is connected to the outlet opening on the cartridge head, whereby a puncturable membrane (242) is provided that closes the rear side of the dispensing plunger in liquid-impermeable manner, and
the receptacle (2, 202) is hollow cylinder-shaped, whereby a porous disk (18, 218), which is permeable to gases and liquids, is provided in the connecting plunger (20, 220) in the receptacle (2, 202), and at least one hollow needle (212) is arranged on the front side of a connecting plunger (20, 220), the at least one hollow needle (212) being connected to the rear side of the porous disk (18, 218) in liquid-permeable manner by means of a channel.

12. Storage and mixing device according to any one of the preceding claims, **characterised in that**
the volume of the monomer liquid (4, 204) in the monomer liquid container (3, 203) is at least as large as the volume of the air-filled intervening spaces between the cement powder particles in the cartridge (1, 201), preferably is at least as large as the volume of the liquid conduits (12, 14, 16, 18, 212, 214, 216, 218) between the internal space of the cartridge (1, 201) and the internal space of the receptacle (2, 202) plus the volume of the air-filled intervening spaces between the cement powder particles in the cartridge (1, 201), and
at least one ventilation opening (90) is arranged in the wall of the receptacle (2, 202), whereby the ventilation opening (90) connects the internal space of the receptacle (2, 202), in which the monomer liquid container (3, 203) is arranged, to the surroundings of the storing and mixing device, whereby the at least one ventilation opening (90) is preferably arranged sufficiently closely in the area of the feed plunger (6, 206) such that it is closed by a motion of the feed plunger (6, 26) in the direction of the front side of the receptacle (2, 202) before the monomer liquid container (3, 203) is opened through the motion of the feed plunger (6, 206).

13. Method for the production of a bone cement dough (54), in particular of a pasty polymethylmethacrylate bone cement dough (54), whereby the bone cement dough (54) is produced from a cement powder (5, 205) and a monomer liquid (4, 204) using a storage and mixing device, whereby the storage and mixing device comprises a receptacle (2, 202) containing a monomer liquid container (3, 203) with the monomer liquid (4, 204) in it and a cartridge (1, 201) containing the cement powder (5, 205), whereby the receptacle (2, 202) is secured or securable to the rear side of the cartridge (1, 201), whereby a closable feedthrough (14, 214) is provided on the rear side of the cartridge (1, 201), **characterised by** the following steps taking place in the order given:
a) inserting the storage and mixing device in an extrusion device (43), whereby the extrusion device (43) comprises a pestle (44) that is propellable in axial direction;
b) propelling a feed plunger (6, 206), supported such as to be mobile in the receptacle (2, 202) on the rear side thereof, in the direction of the cartridge (1, 201) by the pestle (44), whereby the motion of the feed plunger (6, 206) opens the monomer liquid container (3, 203) and presses the monomer liquid (4, 204) from the monomer liquid container (3, 203) through the closable feedthrough (14, 214) into the cartridge (1, 201), whereby the cement powder (5, 205) mixes with the monomer liquid (4, 204) in the internal space of the cartridge (1, 201);
c) separating the receptacle (2, 202) from the cartridge (1, 201), whereby the closable feedthrough (14, 214) is being closed or closes self-actingly during the separation, whereby the cartridge (1, 201) remains in the extrusion device (43) or is reinserted into the extrusion device (43) after the separation;
d) propelling a dispensing plunger (7, 207), supported in the cartridge (1, 201) on the rear side thereof such as to be mobile, in the direction of the front side of the cartridge (1, 201) by the pestle (44), whereby the motion of the dispensing plunger (7, 207) expels the mixture consisting of the cement powder (5, 205) and the monomer liquid (4, 204) from the cartridge (1, 201) in the form of the bone cement dough (54).

14. Method according to claim 13, **characterised in that**
the method is implemented using a storage and mixing device according to any one of the claims 1 to 12.

15. Method according to any one of the claims 13 or 14, **characterised in that**
a first securing means (8, 208) is provided on the rear side of the receptacle (2, 202) and a second securing means (9, 209) is provided on the rear side of the cartridge (1, 201), whereby, during the insertion of the storage and mixing device in step a), the storage and mixing device is secured to a holder (48) of the extrusion device (43) by means of the first securing means (8, 208), and the cartridge (1, 201) is secured to the holder (48) of the extrusion device (43) by the second securing means (9, 209) in step c).

16. Method according to any one of the claims 13 to 15, **characterised in that**
a connecting plunger (20, 220) is arranged on the front side of the receptacle (2, 202), whereby the monomer liquid container (3, 203) is arranged between the connecting plunger (20, 220) and the feed plunger (6, 206), whereby the connecting plunger (20, 220) is driven in the direction of the cartridge (1, 201) in step b), while the feed plunger (6, 206) is propelled and before the monomer liquid (4, 204) is pressed into the cartridge (1, 201), whereby a passage into the cartridge (1, 201) is thus opened such that the internal space of the cartridge (1, 201) and the internal space of the receptacle (2, 202) are connected to each other between the feed plunger (6, 206) and the connecting plunger (20, 220) in liquid-permeable manner.

17. Method according to claim 16, **characterised in that**
by or subsequently to connecting the internal space of the cartridge (1, 201) and the internal space of the receptacle (2, 202) in liquid-permeable manner, the monomer liquid container (3, 203) is opened through a motion of the feed plunger (6, 206) against the connecting plunger (20, 220), in particular a plastic or glass ampoule (3, 203) as the monomer liquid container (3, 203) is crushed between the feed plunger (6, 206) and the connecting plunger (20, 220) such that the monomer liquid (4, 204) is available in the internal space of the receptacle (2, 202) to be pressed into the cartridge (1, 201).

18. Method according to any one of the claims 13 to 17, **characterised in that**
a three-way valve (56, 102), which is operable from outside, is arranged in the extended dispensing opening or downstream from the cartridge (1, 201), whereby the three-way valve (56, 102) is brought into a first position or is in the first position, whereby the three-way valve (56, 102), being in the first position, provides a continuous connection from the internal space of the cartridge (1, 201) through the extended dispensing opening, and an extrusion of the cartridge by means of the extrusion device (43) takes place in step d), whereby the bone cement dough (54) mixed from the cement powder (5, 205) and the monomer liquid (4, 204) is pressed through the three-way valve (56, 102) and through the extended dispensing opening, and a subsequent step e) takes place, in which the three-way valve (56, 102) is transitioned into a second position, whereby the three-way valve (56, 102), being in the second position, stops the flow from the cartridge (1, 201) through the three-way valve (56, 102) and a part of the pressurised bone cement dough (54) downstream from the three-way valve (56, 102) in the extended dispensing opening is pressed through the three-way valve (56, 102) into a collecting container (60, 109), whereby
the three-way valve (56, 102) is moved to the first position again in a step f) after step e) and, by this means, the bone cement dough (54) is guided again through the three-way valve (56, 102) through the dispensing opening, whereby it is preferred for steps d), e), and f) to be repeated once or multiple times in the order given.

## Revendications

1. Dispositif de stockage et de mélange pour produire une pâte de ciment osseux (54) à partir d'un liquide de monomère (4, 204) et d'une poudre de ciment (5, 205) servant de composants de départ du ciment osseux, le dispositif de stockage et de mélange présentant
un réceptacle (2, 202), dans lequel est disposé un récipient de liquide de monomère (3, 203), le liquide de monomère (4, 204) étant contenu dans le récipient de liquide de monomère (3, 203), et
une cartouche (1, 201), dans laquelle est contenue la poudre de ciment (5, 205), où un piston de transport (6, 206) est disposé dans le réceptacle (2, 202), mobile dans la direction longitudinale du réceptacle (2, 202), qui est accessible à partir d'un côté arrière du réceptacle (2, 202), où
un piston d'évacuation (7, 207) est disposé dans la cartouche (1, 201), pouvant être déplacé dans la direction longitudinale à l'intérieur de la cartouche (1, 201), où le côté avant du réceptacle (2, 202) est relié, ou peut être relié, avec le côté arrière de la cartouche (1, 201) de telle manière qu'un espace intérieur du réceptacle (2, 202) est relié avec un espace intérieur de la cartouche (1, 201) en laissant passer le liquide de monomère (4, 204), où la liaison est amovible, et où un premier système de fixation (8, 208) est prévu sur le côté arrière du réceptacle (2, 202) et un deuxième système de fixation (9, 209) est prévu sur le côté arrière de la cartouche (1, 201), où le premier système de fixation (8, 208) et le deuxième système de fixation (9, 209) sont identiques ou du même type, **caractérisé en ce qu'**un passage (14, 214) pouvant être obturé, vers l'espace intérieur de la cartouche (1, 201), est prévu sur le côté arrière de la cartouche (1, 201), en particulier, dans le piston d'évacuation (7, 207) de la cartouche (1, 201).

2. Dispositif de stockage et de mélange selon la revendication 1, **caractérisé en ce que** le passage (14, 214) pouvant être obturé peut être fermé pour ne pas laisser passer de liquide lors du détachement de la liaison permettant le passage du liquide entre le réceptacle (2, 202) et la cartouche (1, 201), où, de manière particulièrement préférée, le passage (14, 214) pouvant être obturé présente une soupape, au moins deux lèvres d'étanchéité (22) ou une membrane (242) se fermant automatiquement pouvant être transpercée.

3. Dispositif de stockage et de mélange selon la revendication 1 ou la revendication 2, **caractérisé en ce**
**qu'**une aiguille creuse (212) ou un petit tube (12) est prévu(e) sur le côté avant du réceptacle (2, 202), avec laquelle ou lequel l'intérieur du réceptacle (2, 202) peut être relié en communication fluidique avec l'espace intérieur de la cartouche (1, 201), où l'aiguille creuse (212), ou le petit tube (12), avec le passage (14, 214) pouvant être obturé, forme la liaison permettant le passage du liquide de monomère (4, 204) vers l'espace intérieur de la cartouche (1, 201) lorsque le réceptacle (2, 202) est relié avec la cartouche (1, 201).

4. Dispositif de stockage et de mélange selon l'une des revendications précédentes, **caractérisé en ce**
**qu'**un piston de connexion (20, 220) est disposé sur le côté avant du réceptacle (2, 202), qui est disposé mobile dans la direction longitudinale à l'intérieur du réceptacle (2, 202), où le récipient de liquide de monomère (3, 203) est disposé entre le piston de connexion (20, 220) et le piston de transport (6, 206), où un passage est prévu dans le piston de connexion (20, 220) qui laisse passer le liquide de monomère (4, 204) et les gaz et grâce auquel une liaison fluidique peut être réalisée vers l'espace intérieur de la cartouche (1, 201), où, de préférence, par un déplacement du piston de transport (20, 220) en direction de la cartouche (1, 201), une cartouche (1, 201) reliée avec le côté avant du réceptacle (2, 202) permet d'ouvrir le passage (14, 214) pouvant être obturé au niveau du côté arrière de la cartouche (1, 201), ou un système de fermeture (12, 212) qui ferme la liaison qui laisse passer le liquide entre l'espace intérieur de la cartouche (1, 201) et l'espace intérieur du réceptacle (2, 202).

5. Dispositif de stockage et de mélange selon l'une des revendications précédentes, **caractérisé en ce que**
le dispositif de stockage et de mélange est un applicateur de ciment osseux permettant le stockage et le mélange des composants de départ et permettant l'application de la pâte de ciment osseux (54) dans la région de la colonne vertébrale, où l'applicateur de ciment osseux présente un orifice de sortie rallongé qui est disposé sur le côté avant de la cartouche (1, 201), où, de manière particulièrement préférée, l'orifice de sortie rallongé est réalisé par un tube de sortie (34, 234), un tuyau (64, 104) et/ou un trocart (66).

6. Dispositif de stockage et de mélange selon la revendication 5, **caractérisé en ce qu'**une soupape à trois voies (56, 102) pouvant être actionnée de l'extérieur est disposée dans l'orifice de sortie rallongé ou entre l'orifice de sortie rallongé et la cartouche (1, 201),
un récipient de récupération (60, 109) est disposé sur la soupape à trois voies (56,102) pour la récupération de pâte de ciment osseux (54), où l'orifice de sortie rallongé débouche dans un orifice d'application, qui est disposé à l'extrémité de l'orifice de sortie rallongé à l'extrémité opposée à la cartouche (1, 201), où la soupape à trois voies (56, 102) est montée de telle manière qu'elle établisse,
dans une première position, une liaison fluidique entre l'orifice d'application et la cartouche (1, 201) et ferme un canal de sortie vers le récipient de récupération (60, 109), et
dans une deuxième position, qu'elle établisse une liaison fluidique entre l'orifice d'application et le récipient de récupération (60, 109) et ferme un passage (61, 119) vers la cartouche (1, 201).

7. Dispositif de stockage et de mélange selon l'une des revendications précédentes, **caractérisé en ce que**
le réceptacle (2, 202) présente un système de connexion (12, 212), notamment, au moins une aiguille creuse (212), ou au moins un petit tube (12), servant de système de connexion (12, 212), où le système de connexion (12, 212) est disposé sur le côté avant du réceptacle (2, 202) et le réceptacle (2, 202) est relié, ou peut être relié, en communication fluidique avec l'espace intérieur de la cartouche (1, 201) par le biais du système de connexion (12, 212) et où le réceptacle (2, 202) est obturé de manière étanche vis-à-vis des liquides jusqu'au système de connexion (12, 212).

8. Dispositif de stockage et de mélange selon l'une des revendications précédentes, **caractérisé en ce que**
le récipient de liquide de monomère (3, 203) est une ampoule en verre (3, 203), une ampoule en matière plastique (3, 203), un pochon en film de matière plastique ou un pochon composite en aluminium et matière plastique qui peut être ouvert(e) à l'intérieur du réceptacle (2, 202), et/ou
la section transversale de l'espace intérieur de la cartouche (1, 121) est au maximum de 4 cm², de préférence, est au maximum de 2,5 cm², de manière particulièrement préférée est au maximum de 1,2 cm².

9. Dispositif de stockage et de mélange selon l'une des revendications précédentes, **caractérisé en ce que**
le premier système de fixation (8, 208) et le deuxième système de fixation (9, 209) sont à fixer au niveau d'un seul support (48) d'un dispositif de pressage (43), notamment d'un pistolet de cartouche.

10. Dispositif de stockage et de mélange selon l'une des revendications précédentes, **caractérisé en ce que**
la cartouche (1, 201) est fermée sur le côté avant avec une tête de cartouche, où un orifice de sortie se trouve dans la tête de cartouche et l'orifice de sortie est fermé avec un obturateur (36, 236) laissant passer le gaz et ne laissant pas passer la poudre de ciment (5, 205) dans la cartouche (1, 201), notamment un filtre poreux (36, 236), où, de préférence, l'obturateur (36, 236) peut être ouvert par une sollicitation en pression axiale ou par l'effet d'une force manuelle, où le réceptacle (2, 202) est fermé ou peut être fermé de manière étanche aux liquides sur le côté arrière par le piston de transport (6, 206), de préférence est fermé ou peut être fermé de manière étanche aux liquides et aux gaz.

11. Dispositif de stockage et de mélange selon l'une des revendications précédentes, **caractérisé en ce que**
la cartouche (1, 201) est en forme de cylindre creux et le deuxième système de fixation (9, 209) est prévu pour la liaison avec un dispositif de pressage (43), où une tête de cartouche ferme le côté avant de la cartouche (1, 201) en forme de cylindre creux, où l'orifice de sortie est disposé dans la tête de cartouche, et où l'orifice de sortie relie le côté extérieur de la tête de cartouche avec le côté intérieur de la tête de cartouche en laissant passer les gaz, où un tube de sortie (34, 234) est raccordé sur la tête de cartouche au niveau de l'orifice de sortie, où une membrane (242) pouvant être transpercée est prévue, laquelle obture le côté arrière du piston d'évacuation (7, 207) en ne laissant pas passer de liquide, et
le réceptacle (2, 202) est en forme de cylindre creux, où un disque (18, 218) poreux laissant passer les gaz et les liquides est prévu dans un piston de connexion (20, 220) dans le réceptacle (2, 202) et au moins une aiguille creuse (212), disposée sur le côté avant d'un piston de connexion (20, 220), est reliée en laissant passer les liquides avec le côté arrière du disque (18, 218) poreux par le biais d'un canal.

12. Dispositif de stockage et de mélange selon l'une des revendications précédentes, **caractérisé en ce que**
le volume du liquide de monomère (4, 204) dans le récipient de liquide de monomère (3, 203) est au moins aussi grand que le volume des espaces intermédiaires remplis d'air entre les particules de poudre de ciment dans la cartouche (1, 201), de préférence, au moins aussi grand que le volume des conduites de liquide (12, 14, 16, 18, 212, 214, 216,218) entre l'espace intérieur de la cartouche (1, 201) et l'espace intérieur du réceptacle (2, 202) plus le volume des espaces intermédiaires remplis d'air entre les particules de poudre de ciment dans la cartouche (1, 201), et
au moins un orifice d'aération (90) est disposé dans la paroi du réceptacle (2, 202), qui relie l'espace intérieur du réceptacle (2, 202), dans lequel est disposé le récipient de liquide de monomère (3, 203), avec l'environnement, où, de préférence, l'au moins un orifice d'aération (90) est disposé de manière si proche dans la région du piston de transport (6, 206) qu'il se ferme par un déplacement du piston de transport (6, 206) en direction du côté avant du réceptacle (2, 202) avant que le récipient de liquide de monomère (3, 203) ne soit ouvert par le déplacement du piston de transport (6, 206).

13. Procédé de fabrication d'une pâte de ciment osseux (54), notamment d'une pâte de ciment osseux en polyméthyl méthacrylate (54) en forme de pâte, où la pâte de ciment osseux (54) est fabriquée à partir d'une poudre de ciment (5, 205) et d'un liquide de monomère (4, 204) avec un dispositif de stockage et de mélange, où le dispositif de stockage et de mélange comprend un réceptacle (2, 202) contenant un récipient de liquide de monomère (3, 203) avec un liquide de monomère (4, 204) à l'intérieur et une cartouche (1, 201) contenant la poudre de ciment (5, 205), où le réceptacle (2, 202) est fixé ou peut être fixé sur le côté arrière de la cartouche (1, 201), où un passage (14, 214) pouvant être obturé est prévu sur le côté arrière de la cartouche (1, 201), **caractérisé par** les étapes suivantes se déroulant les unes après les autres :
a) la mise en place du dispositif de stockage et de mélange dans un dispositif de pressage (43), le dispositif de pressage (43) présentant un poussoir (44) pouvant être actionné axialement,
b) un piston de transport (6, 206) qui est logé mobile à l'intérieur du réceptacle (2, 202) au niveau de son côté arrière est avancé avec le poussoir (44) en direction de la cartouche (1, 201), où le récipient de liquide de monomère (3, 203) est ouvert par le déplacement du piston de transport (6, 206) et le liquide de monomère (4, 204) est pressé hors du récipient de liquide de monomère (3, 203) à travers le passage (14, 214) pouvant être obturé dans la cartouche (1, 201), où la poudre de ciment (5, 205) se mélange avec le liquide de monomère (4, 204) dans l'espace intérieur de la cartouche (1, 201),
c) le réceptacle (2, 202) est séparé de la cartouche (1, 201), où le passage (14, 214) pouvant être obturé est fermé ou se ferme automatiquement lors de la séparation et où la cartouche (1, 201) reste dans le dispositif de pressage (43) ou est insérée dans le dispositif de pressage (43) après la séparation,
d) un piston d'évacuation (7, 207) qui est logé mobile à l'intérieur de la cartouche (1, 201) au niveau de son côté arrière, est avancé avec un poussoir (44) en direction du côté avant de la cartouche (1, 201), où le mélange à base de la poudre de ciment (5, 205) et du liquide de monomère (4, 204) est expulsé de la cartouche (1, 201) sous forme de pâte de ciment osseux (54) par le déplacement du piston de transport (6, 206).

14. Procédé selon la revendication 13, **caractérisé en ce que**
le procédé est réalisé avec un dispositif de stockage et de mélange selon l'une des revendications 1 à 12.

15. Procédé selon l'une des revendications 13 ou 14, **caractérisé en ce**
**qu'**un premier système de fixation (8, 208) est prévu sur le côté arrière du réceptacle (2, 202) et un deuxième système de fixation (9, 209) est prévu sur le côté arrière de la cartouche (1, 201), où, lors de l'emploi du dispositif de stockage et de mélange dans l'étape a), le dispositif de stockage et de mélange est fixé à un support (48) du dispositif de pressage (43) par le biais du deuxième système de fixation (9, 209), et dans l'étape c), la cartouche (1, 201) est fixée sur le support (48) du dispositif de pressage (43) avec le premier système de fixation (8, 208).

16. Procédé selon l'une des revendications 13 à 15, **caractérisé en ce**
**qu'**un piston de connexion (20, 220) est disposé sur le côté avant du réceptacle (2, 202), où le récipient de liquide de monomère (3, 203) est disposé entre le piston de connexion (20, 220) et le piston de transport (6, 206), où, dans l'étape b), lors de l'avancée du piston de transport (6, 206) avant la poussée du liquide de monomère (4, 204) dans la cartouche (1, 201), le piston de connexion (20, 220) est entraîné en direction de la cartouche (1, 201), où, ce faisant, un passage est ouvert dans la cartouche (1, 201), de sorte que l'espace intérieur de la cartouche (1, 201) et l'espace intérieur du réceptacle (2, 202) entre le piston de transport (6, 206) et le piston de connexion (20, 220) sont reliés l'un à l'autre dans une liaison fluidique.

17. Procédé selon la revendication 16, **caractérisé en ce que**,
lors ou après l'établissement de la liaison permettant le passage de liquide de l'espace intérieur de la cartouche (1, 201) et de l'espace intérieur du réceptacle (2, 202), le récipient de liquide de monomère (3, 203) est ouvert par un déplacement du piston de transport (6, 206) contre le piston de connexion (20, 220), en particulier, une ampoule en matière plastique ou en verre (3, 203) servant de récipient de liquide de monomère (3, 203), est écrasée entre le piston de transport (6, 206) et le piston de connexion (20, 220), de sorte que le liquide de monomère (4, 204) est disponible dans l'espace intérieur du réceptacle (2, 202) pour être pressé dans la cartouche (1, 201).

18. Procédé selon l'une des revendications 13 à 17, **caractérisé en ce**
**qu'**une soupape à trois voies (53, 102) pouvant être actionnée de l'extérieur est disposée dans l'orifice de sortie rallongé ou derrière la cartouche (1, 201), où la soupape à trois voies (53, 102) est mise dans une première position ou se trouve dans la première position, où la soupape à trois voies (56, 102) permet une liaison de part en part de l'espace intérieur de la cartouche (1, 201) à travers l'orifice de sortie rallongé et un pressage de la cartouche (1, 201) a lieu dans l'étape d) à l'aide du dispositif de pressage (43), où la pâte de ciment osseux (54) mélangée à partir de la poudre de ciment (5, 205) et du liquide de monomère (4, 204) est pressée à travers la soupape à trois voies (56, 102) et à travers l'orifice de sortie rallongé et qu'une étape e) ultérieure se produit **en ce que** la soupape à trois voies (56, 102) est transférée dans une deuxième position, où la soupape à trois voies (56, 102) arrête l'écoulement sortant de la cartouche (1, 201) dans la deuxième position par la soupape à trois voies (56, 102) et une partie de la pâte de ciment osseux (54) se trouvant sous pression derrière la soupape à trois voies dans l'orifice de sortie rallongé est pressée dans un récipient de récupération (60, 109) par la soupape à trois voies (56, 102), où après l'étape e), dans une étape f), la soupape à trois voies (56, 102) est de nouveau transférée dans la première position et ainsi la pâte de ciment osseux (54) est à nouveau menée à travers l'orifice de sortie rallongé, où, de préférence, les étapes d), e) et f) sont ensuite effectuées chronologiquement répétées une ou plusieurs fois.
